# EUROPÄISCHE PATENTSCHRIFT

(11) **EP 1 169 441 B1**
(45) Veröffentlichungstag und Bekanntmachung des Hinweises auf die Patenterteilung: **08.06.2005**
(21) Anmeldenummer: 00916980.6
(22) Anmeldetag: 20.03.2000
(51) Int. Cl.: C12N 15/12, A61K 38/17, A61K 48/00, C07K 14/47, A61P 9/10

(54) **Pharmazeutische Zusammensetzungen zur Therapie der Herzinsuffizienz**
Pharmaceutical compositions for the treatment of heart failure
Compositions pharmaceutiques pour le traitement d'une insuffisance cardiaque

(30) Priorität: 07.04.1999 DE 19915485
(43) Veröffentlichungstag der Anmeldung: 09.01.2002
(73) Patentinhaber: Katus, Hugo A., 23909 Ratzeburg (DE); Remppis, Andrew, 23564 Lübeck (DE)
(72) Erfinder: Katus, Hugo A., 23909 Ratzeburg (DE); Remppis, Andrew, 23564 Lübeck (DE)
(74) Vertreter: UEXKÜLL & STOLBERG
(86) Internationale Anmeldenummer: PCT/EP2000/002453
(87) Internationale Veröffentlichungsnummer: WO 2000/061742

(56) Entgegenhaltungen:
- WO-A-97/14427
- WO-A-98/50079
- US-A- 5 849 528
- REMPPIS A ET AL: "Altered expression of the Ca(2+)-binding protein S100A1 in human cardiomyopathy." BIOCHIMICA ET BIOPHYSICA ACTA, (1996 OCT 11) 1313 (3) 253-7. , XP000953069
- HE T -C ET AL: "A simplified system for generating recombinant adenoviruses" PROCEEDINGS OF THE NATIONAL ACADEMY OF SCIENCES OF USA,NATIONAL ACADEMY OF SCIENCE. WASHINGTON,US, Bd. 95, März 1998 (1998-03), Seiten 2509-2514, XP002144832 ISSN: 0027-8424 in der Anmeldung erwähnt
- SCHAFER B W ET AL: "The S100 family of EF-hand calcium-binding proteins: functions and pathology" TIBS TRENDS IN BIOCHEMICAL SCIENCES,EN,ELSEVIER PUBLICATION, CAMBRIDGE, Bd. 21, Nr. 4, 1. April 1996 (1996-04-01), Seiten 134-140, XP004050923 ISSN: 0968-0004 in der Anmeldung erwähnt
- MOST PATRICK ET AL: "The hydrophobic epitopes and the linker region of the EF-hand Ca-binding protein S100A1 enhance the caffeine induced Ca-release from SR." CIRCULATION, Bd. 110, Nr. 18 SUPPL., 2. November 1999 (1999-11-02), Seite I.420 XP000953109 72nd Scientific Sessions of the American Heart Association;Atlanta, Georgia, USA; November 7-10, 1999 ISSN: 0009-7322
- PLEGER S T ET AL: "Increased heart function by S100A1 gene therapy." EUROPEAN HEART JOURNAL, Bd. 20, Nr. ABSTR. SUPPL., August 1999 (1999-08), Seite 374 XP000952821 XXIst Congress of the European Society of Cardiology;Barcelona, Spain; August 28-September 1, 1999 ISSN: 0195-668X
- MOST PATRICK ET AL: "S100A1 adenoviral gene transfer leads to an increased velocity of shortening and relengthening in adult rat cardiomyocytes." CIRCULATION, Bd. 110, Nr. 18 SUPPL., 2. November 1999 (1999-11-02), Seite I.420 XP002152357 72nd Scientific Sessions of the American Heart Association;Atlanta, Georgia, USA; November 7-10, 1999 ISSN: 0009-7322
- FEBS LETTERS, Bd. 497, 2001, Seiten 95-98,
- PROC. NATL. ACAD. SCI., USA, Bd. 98, 2001, Seiten 13889-13894,

## Beschreibung

Die vorliegende Erfindung betrifft Arzneimittel zur Behandlung von Herzinsuffizienz, die eine therapeutisch wirksame Menge eines oder mehrerer S100-Proteine, einer oder mehrerer Mutanten oder Fragmente derselben oder für diese Aminosäuresequenzen kodierende Nukleinsäuresequenz(en), gegebenenfalls in einen oder mehrere Gentransfervektoren integriert, enthalten.

Arzneimittel zur Behardlung der Herzinsuffizienz durch Verwendung von zum Beispiel einen Fibroblasten-Wachstrumsfaktor sind in Stand der Technik bekannt Siehe WO 98/50079.

Veränderungen der intrazellulären Ca²⁺-Homöostase spielen im Rahmen der Herzinsuffizienz auf molekularer Ebene eine pathophysiologisch grundlegende Rolle. Gwathmey at al (1) konnten als erste an kontrahierenden Herzmuskelpräparaten von Patienten mit terminaler Herzinsuffizienz verlängerte intrazelluläre Ca²⁺-Transienten nachweisen. Vor dem Hintergrund erhöhter diastolischer Ca²⁺-Spiegel und verminderter systolischer Ca²⁺-*peaks* (2) wurde dieser Befund als Hinweis auf eine Dysfunktion des sarkoplasmatischen Retikulums (im folgenden mit SR abgekürzt) interpretiert, der auf hämodynamischer Ebene mit einer inversen Kraft-Frequenz-Beziehung des myopathischen Herzmuskels korreliert (3).

Von zentraler Bedeutung ist hierbei die verminderte Rückaufnahme von Ca²⁺ in das SR während der Diastole durch die Ca²⁺ATPase (Ca²⁺ Pumpe des SR, die das Ca²⁺ aus dem Zytosol in das SR gegen einen Konzentrationsgradienten von 1:10000 pumpt). Diese führt einerseits zu einer gestörten Relaxation des Herzmuskels während der Diastole sowie zu einer damit verbundenen Verminderung der Ca²⁺-Ausschüttung während der Systole und so zu einer verminderten Kraftentfaltung des Herzmuskels. Unter anderem liegt dieser Beobachtung die Tatsache zugrunde, daß insuffiziente Herzen verminderte cAMP Spiegel aufweisen (4). Denn die cAMP abhängige Phosphorylierung von Phospholamban ist Voraussetzung zur Aktivierung der Ca²⁺ATPase des SR.

Eine der bisherigen Strategien, die Kontraktilität zu verbessern, zielte daher auf eine Erhöhung intrazellulärer cAMP Spiegel durch die Gabe von Phosphodiesterasehemmern. Obwohl dieser pharmakologische Ansatz kurzfristig zu einer Verbesserung der Herzleistung führt, wurde diese Therapieoption für die chronische Behandlung der Herzinsuffizienz verlassen, da sie im Vergleich zu Placebo zu einer Übersterblichkeit der untersuchten Patienten von 53% führte (5, 6).

Eine weitere bislang verfolgte Strategie besteht darin, das verminderte Ca²⁺-Angebot während der Systole durch eine Sensibilisierung des kontraktilen Apparates durch Ca²⁺ *sensitizer* effektiver zu nutzen, die bei gleicher Ca²⁺-Konzentration eine höhere Kraftentfaltung des kontraktilen Apparates erlauben. Bisher durchgeführte klinische Studien zu Pimobendan enttäuschten jedoch, da gegenüber Plazebo keine signifikante Verbesserung der Herzfunktion zu dokumentieren war (7). Für die inhomogene Gruppe neuer Ca²⁺ *sensitizer* liegen noch keine klinischen Daten vor. Dieser therapeutische Ansatz führt durch eine erhöhte Ca²⁺ Sensitivität des kontraktilen Apparates jedoch bei einigen Ca2⁺ sensitizern zu einer verschlechterten Relaxation, so daß der therapeutische Vorteil einer verstärkten systolischen Kraftentfaltung durch eine Störung der Diastole in Frage gestellt wird (8).

Bei der Suche nach weiteren molekularen Ursachen für eine eingeschränkte Funktion des SR im Rahmen der Herzinsuffizienz konnte für verschiedene Tiermodelle wie auch für den Menschen eine verminderte Ca²⁺ATPase Aktivität in grob gereinigten Membranpräparaten (crude membranes) insuffizienter Herzen gemessen werden, so daß eine veränderte Proteinzusammensetzung des SR als Ursache vermutet wurde. Untersuchungen zur Genexpression der SR-Proteine Phospholamban, Ca²⁺ATPase und Ryanodin-Rezeptor erbrachten jedoch unterschiedliche und zum Teil widersprüchliche Daten. So fanden verschiedene Arbeitsgruppen (9, 10, 11) eine signifikante Minderexpression der für Phospholamban und Ca²⁺ATPase kodierenden Gene auch auf Proteinebene, während Movsesian et al (12) und Schwinger et al (13) keine signifikanten Expressionsunterschiede dokumentieren konnten und Arai et al (14) eine differentielle Expression im Verlaufe der Hypertrophieentwicklung fand. Obwohl an grob gereinigten Membranpräparationen terminal insuffizienter Herzen eine signifikant niedrigere Ca²⁺ATPase Aktivität nachgewiesen wurde (15), konnte dieser Befund an hochgereinigten Membranpräparaten des sarkoplasmatischen Retikulums nicht nachvollzogen werden (12). Bislang wurden die widersprüchlichen Ergebnisse auf unterschiedliche Analysemethoden zurückgeführt (12)

Aufgabe der vorliegenden Erfindung ist es daher, Arzneimittel zur Behandlung der Herzinsuffizienz, insbesondere der Behandlung bzw. Verbesserung der im Rahmen der Herzinsuffizienz eingeschränkten Pumpleistung des Herzens, zur Verfügung zu stellen. Die Arzneimittel sollen vorzugsweise ferner die Herzleistung insgesamt erhöhen und sich für eine Behandlung der akuten und chronischen Herzinsuffizienz eignen.

Erfindungsgemäß wird diese Aufgabe allgemein durch Verwendung von S100-Proteinen gelöst, wobei die Proteine entweder unmittelbar als Wirkstoff eingesetzt werden oder durch einen gentherapeutischen Ansatz eine Überexpression von S100-Proteinen im Herzmuskel bewirkt wird. Im besonderen dienen zur Lösung der Aufgabe die Gegenstände der Ansprüche 1 bis 35.

S100 Proteine gehören ebenso wie Calmodulin zur Gruppe Ca²⁺-bindender Proteine mit EF-Hand Ca²⁺-Bindungsmotiv, von denen inzwischen über 200 bekannt sind. Die Familie der S100 Proteine selbst umfaßt 17 Mitglieder, von denen 13 auf einem engen Gencluster auf Chromosom 1q21 kodiert sind. Funktionell sind diese Proteine in die Regulation der Zelldifferenzierung, der Zellzyklusregulation, der Signaltransduktion sowie der Ca²⁺-Homöostase eingebunden (16). Im Gegensatz zum ubiquitär exprimierten Calmodulin weisen S100 Proteine ein gewebsspezifisches Expressionsmuster auf (17). Sie übersetzen daher das Ca²⁺-Signal in eine gewebsspezifische Antwort, indem sie nach Binden von Ca²⁺ an ihren EF-Händen mit spezifischen Zielproteinen wechselwirken. (16). S100 Proteine weisen eine stark konservierte Aminosäuresequenz mit hoher Homologie innerhalb der S100-Familie auf. Die Aminosäure- und cDNA-Sequenzen sind im Sequenzprotokoll als SEQ ID NO: 1 bis 30 (Numerische Kennzahl <210>) dargestellt.

Unter S100-Proteinen im Sinne der Erfindung sind die kompletten nativen Proteine, Mutanten der S100-Proteine, Peptide (Fragmente) der S100-Proteine oder Peptid-Mutanten (mit einer Homologie von mindestens 60 %, vorzugsweise mindestens 90 % und besonders bevorzugt mindestens 95 %) sowie rekombinant hergestellte Proteine bzw. Peptide oder Mutanten oder synthetische Peptide bzw. Mutanten zu verstehen. Gemäß einer besonderen Ausführungsform der Erfindung ist das S100-Protein S100β (S100B), S100A1, S100A2, S100A4 oder S100A6 (vgl. Schäfer et al., Genomics 23 (1995) 638-643). Im folgenden werden unter dem Begriff "S100-Protein" oder "Protein" alternativ auch die genannten Mutanten oder Peptide verstanden. Aufgrund der Spezies-übergreifenden Homologie der S100-Proteine können erfindungsgemäß Proteine und dafür kodierende Sequenzen beliebiger Spezies (wie z.B. Schwein, Rind etc.) eingesetzt werden, wobei die entsprechenden humanen Sequenzen am meisten bevorzugt sind.

Gegenstand der vorliegenden Erfindung sind daher Arzneimittel (pharmazeutische Präparate) zur Behandlung von Herzerkrankungen mit verminderter Kontraktionskraft gleich welcher Ursache (primäre und sekundäre Kardiomyopathien), die eine therapeutisch wirksame Menge eines oder mehrerer S100A1-S100A13-Proteine (vorzugsweise, S100A1, S100A2, S100A4 oder S100A6), einer oder mehrerer Mutanten oder Fragmente derselben enthalten, die gegebenenfalls mit pharmazeutisch verträglichen Hilfs- und/oder Trägerstoffen formuliert ist.

Gemäß bevorzugten Ausführungsformen der Erfindung ist das S100-Protein S100A1, das die in SEQ ID NO: 2 gezeigte Aminosäuresequenz aufweist, und die bevorzugten Fragmente weisen die in SEQ ID NO: 32, 34 und 36 gezeigten Aminosäuresequenzen auf. Überwiegend hydrophobe Peptide können zur Verbesserung der Löslichkeit C- oder N-terminal mit hydrophilen Aminosäuren verlängert sein. Vorzugsweise werden erfindungsgemäß die trunkierten bzw. modifizierten Fragmente gemäß SEQ ID NO: 37, 38 und/oder 39 eingesetzt.

Als Wirkstoffe dienen entweder einzelne der genannten Proteine, Mutanten oder Peptide, es können jedoch auch beliebige Mischungen derselben verwendet werden, wie z.B. eine Kombination von mindestens zwei der in SEQ ID NO: 32, 34, 36, 37, 38 und 39 dargestellten Peptide.

Im Rahmen der vorliegenden Erfindungen werden unter primären Kardiomyopathien familiär vererbte Kardiomyopathien und Kardiomyopathien auf dem Boden von Spontanmutationen verstanden, unter sekundären Kardiomyopathien werden ischämische Kardiomyopathie auf dem Boden einer Arteriosklerose, dilatative Kardiomyopathie auf dem Boden einer infektiösen oder toxischen Erkrankung des Myokards, hypertensive Herzerkrankung auf dem Boden einer pulmonalarteriellen und/oder einer arteriellen Hypertonie, strukturelle Herzerkrankungen auf dem Boden von Rhythmusstörungen und Erkrankungen der Herzklappen verstanden, wobei die primären und sekundären Kardiomyopathien Ursache der Herzinsuffizienz sind. Die vorliegende Anmeldung betrifft daher ferner Arzneimittel zur Behandlung von Herzinsuffizienz.

Zur Applikation ist eine direkte Injektion von gereinigtem S100 Protein (bzw. Mutante oder Peptid) entweder intravenös, intraarteriell oder intrakoronar bzw. langfristig auch die orale Gabe von rekombinantem Protein oder von synthetischen Peptidanaloga möglich.

Im Rahmen der vorliegenden Erfindung wurde überaschenderweise gefunden, daß im Zellkulturmodell nach Behandlung mit S100-Protein, insbesondere S100A1-Protein, sowie nach S100-Genaddition (insbesondere S100A1) eine Zunahme der Verkürzungs- und Relaxationsgeschwindigkeit von kultivierten Myokardzellen zu dokumentieren ist, die mit veränderten intrazellulären Ca²⁺-Transienten im Sinne einer erhöhten systolischen Ca²⁺-Freisetzung aus dem SR und einer beschleunigten Ca²⁺-Wiederaufnahme in das SR korreliert (siehe Beispiele). Diese Ergebnisse ließen sich auch mit anderen Proteinen der S100-Familie bestätigen.

Die Erfindung betrifft daher ferner Arzneimittel zur Behandlung von primären und sekundären Kardiomyopathien sowie von Herzinsuffizienz, die für ein oder mehrere S100-Proteine oder eine oder mehrere Mutanten oder Fragmente derselben kodierende Nukleinsäuresequenz(en) enthalten, wobei die Nukleinsäure(n) gegebenenfalls mit pharmazeutisch verträglichen Hilfs- und/oder Trägerstoffen formuliert ist (sind). Diese Nukleinsäuresequenz(en) kann (können) auch in einem oder mehreren Gentransfervektoren enthalten sein. Die für die erfindungsgemäß verwendeten S100-Proteine kodierenden Nukleinsäuresequenzen sind in SEQ ID NO: 1 bis 35 wiedergegeben, wobei SEQ ID NO: 1 (S100A1 cDNA) besonders bevorzugt ist. Gemäß einer bevorzugten Ausführungsform der Erfindung verwendet man eine Kombination von mindestens zwei der für SEQ ID NO: 32, 34, 36, 37, 38 und 39 kodierenden Nukleinsäuresequenzen (insbes. SEQ ID NO: 31, 33, 35 sowie für SEQ ID NO: 37, 38 und 39 kodierende Sequenzen). Im gentherapeutischen Ansatz kommen dabei ein oder mehrere diese Kombination umfassende Gentransfervektoren in Betracht, d.h. die genannten Nukleinsäuresequenzen können entweder in mehrere Vektoren einkloniert sein (z.B. jeweils einzeln), oder die Kombination der kodierenden Sequenzen kann in einem einzigen Vektor enthalten sein.

Die Nukleinsäuren oder Gentransfervektoren sind gegebenenfalls für die intravenöse, intraarterielle, intrakoronare oder orale Applikation formuliert. Eine Verwendung von DNA in liposomalen Fraktionen stellt eine weitere Möglichkeit der Applikation dar, auch wenn sie eine geringere Transfektionseffizienz aufweist.

Gegenstand der vorliegenden Erfindung ist somit ferner die Verwendung eines Konstruktes, das eine Überexpression von S100, vorzugsweise von S100A1, im Herzmuskel ermöglicht. Bevorzugt ist hierbei ein virales Konstrukt, das die DNA eines S100-Proteins, insbesondere des S100β-, S100A1-, S100A2, S100A4- und des S100A6-Proteins, enthält und das vorzugsweise über einen Koronarkatheter koronar appliziert wird, wobei es nach dieser Applikation die höchste Transfektionseffizienz aufweist.

Die vorliegende Erfindung betrifft somit ferner ein Verfahren zur Herstellung eines für ein oder mehrere S100-Proteine oder ein oder mehrere Mutanten oder Fragmente derselben kodierenden Gentransfervektor, bei dem man eine für das (die) Protein(e), die Mutante(n) oder das (die) Fragment(e) kodierende(n) Nukleinsäuresequenz(en) in einen oder mehrere zur Gentherapie geeignete Vektoren kloniert.

Die kodierende Nukleinsäuresequenz wird vorzugsweise aus der Gruppe der in SEQ ID NO: 1 bis 30 dargestellten Nukleinsäuresequenzen ausgewählt. Gemäß einer besonders bevorzugten Ausführungsform der Erfindung verwendet man zur Herstellung der Gentransfervektoren oder zur unmittelbaren Applikation die S100 DNA (für S100A1 kodierende Sequenz gemäß SEQ ID NO: 1), oder die für die Fragmente gemäß SEQ ID NO 32, 34, 36, 37, 38 und/oder 39 kodierenden Nukleinsäuresequenzen (insbes. SEQ ID NO: 31, 33, 35 sowie für SEQ ID NO: 37, 38 und 39 kodierende Sequenzen).

Prinzipiell sind mehrere virale Vektorsysteme geeignet, um eine Transfektion des Herzgewebes mit S100 DNA vorzunehmen. Neuere Vektorsysteme (G. Bilbao et al., FASEB J. 11 (1977) 624-634; A. Amalficano et al., J. Virol. 72 (1998) 926-933) können aufgrund nur noch minimaler immunologischer Nebenwirkungen gegenüber bekannten adenoviralen Vektoren eine Verbesserung der gentherapeutischen Effizienz erbringen. Die gentherapeutische Effizienz kann darüberhinaus durch Verwendung starker herzspezifischer Promotoren wie z.B. durch einen α-Myosin-Schwerkettenpromotor optimiert werden.

Die Wirkung der S100-Proteine kann ferner durch Verwendung entsprechender Sense-Oligonukleotide verstärkt werden, es kann aber auch der Einsatz von anti-Sense-Oligonukleotiden für S100-inhibitorische Substanzen in Betracht gezogen werden. Sense-Oligonukleotide (bzw. anti-Sense-Oligonukleotide von antagonistisch wirkenden Proteinen/Peptiden) bzw. S100-Proteine können direkt in die Gefäßwand der Koronararterien transferiert werden. Unter Anwendung eines Ballonsystems mit einer Geloberfäche als Trägersubstanz für Oligonukleotide bzw. Proteine wird der Transfer dieser Moleküle in die Endothelschicht des Gefäßes unter Inflation des Ballons erreicht. Diese Anwendungsmethode beschränkt sich jedoch auf die Beeinflussung der kardialen Kontraktilität durch Verbesserung der Gefäßfunktion im Sinne einer Verbesserung der Endothel- bzw. der glattmuskulären Funktion, da hiermit nur eine lokale Erhöhung der S100-Proteinkonzentration erreicht werden kann.

Die Transfektion mit S100A1 DNA führt zu einer erhöhten Konzentration von S100A1-Protein im Herzgewebe, wobei so gentherapeutisch eine im Rahmen der Herzinsuffizienz auftretende Minderexpression von S100A1 ursächlich behandelt wird und die Funktion dieses Proteins wieder gewährleistet ist. Hervorzuheben ist die positiv inotrope und lusitrope Wirkung von S100A1 im Herzgewebe. Unter S100A1 Gentherapie von kultivierten Myokardzellen, von rekonstituiertem Herzgewebe (18), sowie in einem in vivo-Modell des Hasenherzens zeigt sich nach Überexpression von S100A1 eine signifikante Steigerung der Kontraktionsgeschwindigkeit um mindestens 20% (positive Inotropie) sowie eine beschleunigte Relaxation (positive Lusitropie). Dieser Effekt beruht auf einer verbesserten Funktion des sarkoplasmatischen Retikulums, die durch eine beschleunigte sowie vermehrte Ca²⁺-Freisetzung aus dem SR sowie Wiederaufnahme von Ca²⁺ zurück in das SR gekennzeichnet ist. Belegt ist dies in eigenen bislang unveröffentlichten Versuchen mit bioluminiszenz-gestützter Analyse der intrazellulären Ca2+ Transienten (siehe Beispiele).

Der Vorteil dieser Erfindung besteht insbesondere darin, daß die für die Herzinsuffizienz pathognomonische Dysfunktion des SR ursächlich behandelbar wird.

Im Rahmen der vorliegenden Erfindung werden somit nicht nur Arzneimittel zur Behandlung (chronischer) Herzinsuffizienz zur Verfügung gestellt, sondern auch Arzneimittel zur Behandlung von Herzerkrankungen, die als Ursache einer Herzinsuffizienz verstanden werden, wie primäre Kardiomyopathien (z.B. familiär vererbte Kardiomyopathien, Kardiomyopathien auf dem Boden von Spontanmutationen) und sekundäre Kardiomyopathien (z.B. ischämische Kardiomyopathie auf dem Boden einer Arteriosklerose, dilatative Kardiomyopathie auf dem Boden einer infektiösen oder toxischen Erkrankung des Myokards, hypertensive Herzerkrankung auf dem Boden einer pulmonalarteriellen und/oder arteriellen Hypertonie, strukturelle Herzerkrankungen auf dem Boden von Rhythmusstörungen, Erkrankungen der Herzklappen etc.).

Ein weiterer Vorteil dieser Erfindung besteht in der Tatsache, daß weitere Funktionen für S100, insbesondere für S100A1, beschrieben sind, die den gentherapeutischen Ansatz zur Behandlung der Herzinsuffizienz wesentlich erweitern können.

Untersuchungen von Donato sowie von Garbuglia et al (19, 20) zeigten eine Inhibition der Polymerisation von Mikrotubuli sowie von intermediären Filamenten durch S100A1 in biochemischen Rekonstituierungsassays. Immunhistochemische Analysen von Schaper et al (21) dokumentieren eine signifikante Zunahme des mikrotubulären Netzwerkes sowie ungeordneter intermediärer Filamente in explantierten Herzen von Patienten mit Herzinsuffizienz des Schweregrades NYHA IV (New York Heart Association IV), die zu einer erhöhten viscösen Last des insuffizienten Myokards und damit zu einer verminderten Kontraktionsgeschwindigkeit führt (22). Die therapeutische Überexpression von S100A1 kann somit eine überschießende Zytoskelettvernetzung verhindern und die Kontraktionsbedingungen durch Absenken der viscösen Last verbessern.

Daten von Baudier et al (23) belegen schließlich eine Inhibition der Proteinkinase C durch S100A1. Da die Aktivierung der Proteinkinase C eine Schlüsselstellung in der Signaltransduktionskaskade der zur Insuffizienz führenden Hypertrophieprozesse hat (24), könnte eine Inhibition der Proteinkinase C durch erhöhte S100A1 Gewebsspiegel im insuffizienten Myokard von großer therapeutischer Bedeutung für eine Normalisierung der Signaltransduktion des insuffizienten Herzens sein.

Die nachfolgenden Beispiele, Figuren und das Sequenzprotokoll sollen die Erfindung näher erläutern, ohne sie darauf zu beschränken.

### Beispiel 1

### Reinigung von rekombinantem S100A1

Humanes rekombinantes S100A1 wurde in gentechnisch veränderten E. coli hergestellt. Dazu wurde die cDNA von S100A1 in einen pGEMEX-Expressionsvektor kloniert, kompetente E. coli wurden mit diesem Vektor transformiert und über eine im Vektor enthaltene Ampicillinresistenz selektioniert. Die gentechnisch veränderten Bakterien wurden bis zu ihrer Verwendung in 50 % Glycerin bei -80 °C gelagert und dann nach folgendem Schema angesetzt: 3 ml LB-Medium mit 100 µg/ml Ampicillin wurden aus dem Vorratsgefäß mit einer Impföse beimpft und über Nacht bei 37 °C inkubiert. Am nächsten Tag wurde die Übernachtkultur zu 250 ml LB-Medium mit 100 µg/ml Ampicillin und 30 µg/ml Chloramphenicol geben. Die optische Dichte wurde alle 30 Minuten gemessen, ab OD > 0,5 wurden 120 µl 1 M IPTG hinzugeben. Nach weiteren 3-4 Stunden wurde das Medium abzentrifugiert und die Pellets 3-4 Mal aufgetaut und wieder eingefroren um die Bakterien zu lysieren.

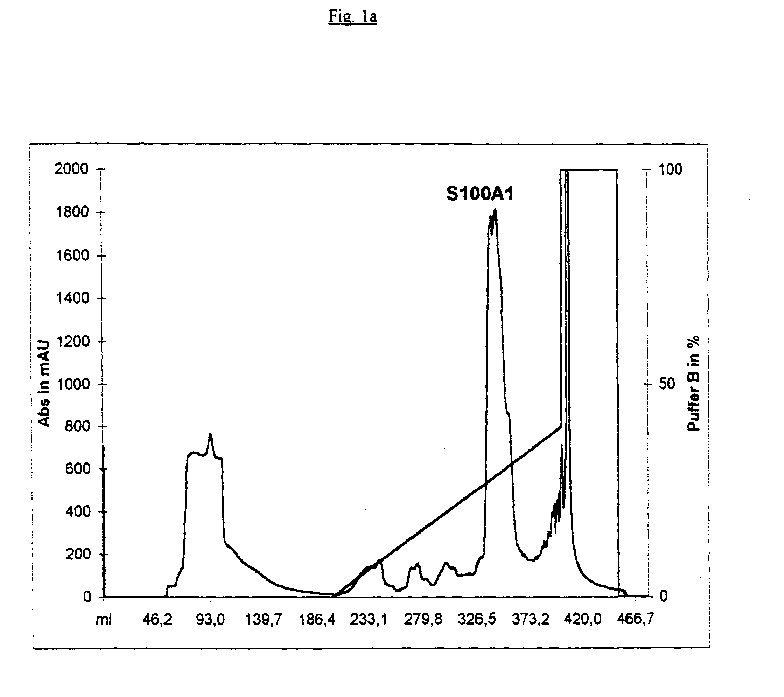

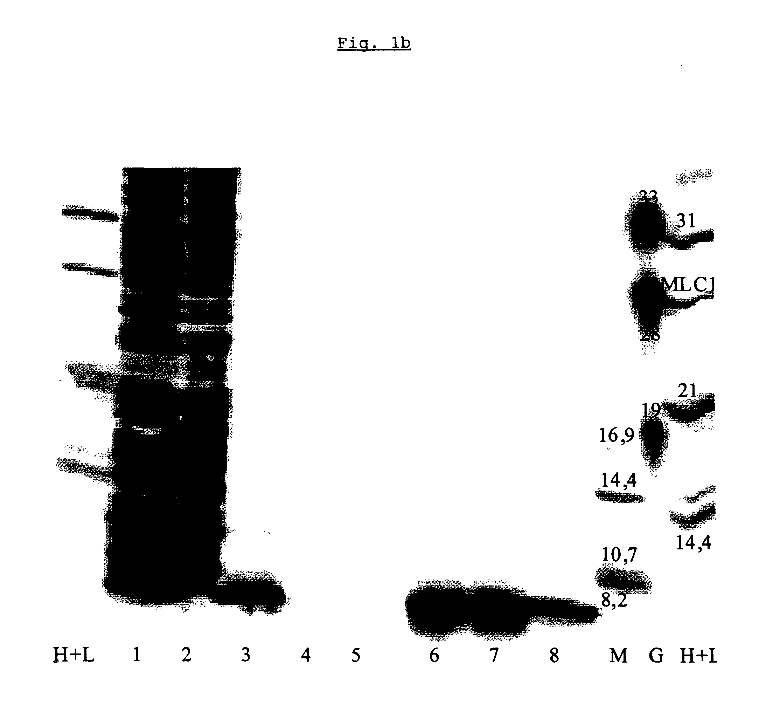

Die bakteriellen Pellets wurden in Extraktionspuffer (25 mM Tris-HCl, pH 7,5, 50 mM KCl, 1 mM PMSF, 5 mM EDTA) mit Hilfe von Ultraschall homogenisiert. Die Überstände wurden mit gesättigter Ammoniumsulfatlösung auf eine Endkonzentration von 2 M gebracht und abzentrifugiert. Der Überstand der Ammoniumsulfatfällung wurde auf eine HiTrap Octyl-Sepharose-Säule (Fa. Phamacia, Freiburg) appliziert, die vorher mit Puffer A (25 mM Tris-HCl, pH 7,5, 2 mM CaCl2) äquilibriert wurde. Das an die Säule gebundene S100A1 wurde mit einem Stufengradienten auf Puffer B (25 mM Tris-HCl, pH 9,5, 5 mM EGTA) eluiert. Um eine Reinheit von mehr als 95 % zu erlangen, wurde das S100-Eluat anschließend einer Anionenaustauscherchromatographie unterzogen. Die Probe wurde auf eine zuvor in Puffer A (25 mM Tris-HCl, pH 7,5) äquilibrierte HiTrapQ Säule (Fa. Pharmacia) aufgetragen und durch einen linearen Gradienten auf 40 % Puffer B (25 mM Tris-HCl, pH 7,5, 1 M NaCl) eluiert. Das gereinigte S100A1 wurde in einem Vakuumverdampfer einkonzentriert und gegen 10 mM HEPES (pH 7,5) dialysiert und schließlich aliquotiert bei -80 °C gelagert. Fig. 1a zeigt das Elutionsprofil der HiTrapQ Säule sowie ein SDS Polyacrylamidgel und Fig. 1b den dazugehörigen Western Blot, die zur Qualitätskontrolle der S100A1 Aufreinigung durchgeführt wurden. Man erkennt, daß das verwendete Reinigungsverfahren zu einem hohen Reinheitsgrad des S100A1 Proteins führt.

### Beispiel 2

### Gesteigerte intrazelluläre Ca²⁺ Transienten in kultivierten neonatalen Rattenkardiomyozyten nach Kokultivation mit rekombinantem S100A1

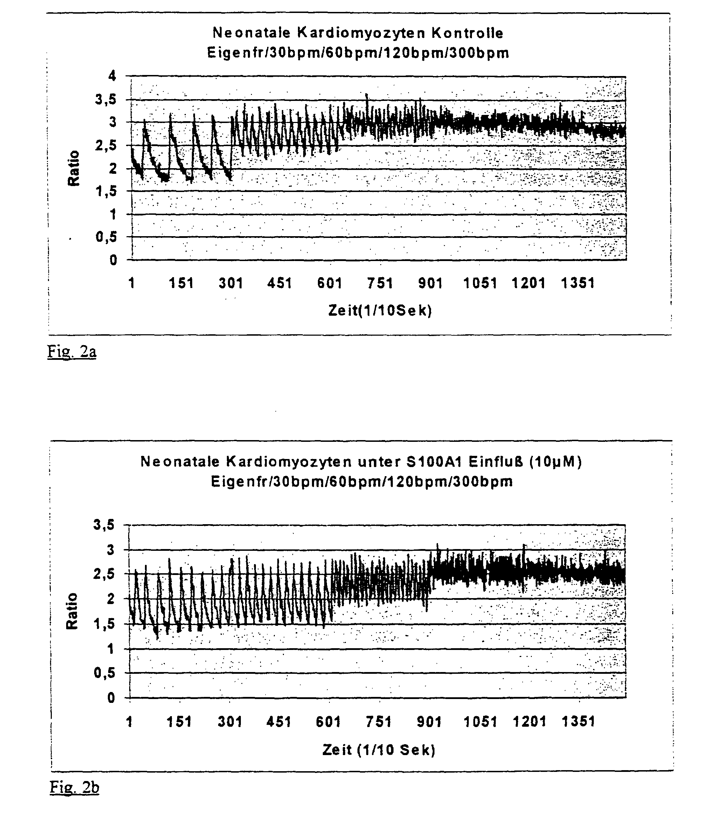

Neonatale Rattenherzen wurden 3 Tage alten Ratten entnommen, Ventrikel von den Atrien getrennt und grob zerkleinert in ADS Puffer (6,8g NaCl, 4,76g HEPES, 0,12 g NaH₂PO₄, 1g Glucose, 0,4g KCL, 0,1g MgSO₄ ad 1000 ml H₂O) auf Eis gelagert. In einem Bioreaktor (Wheaton® Becher) wurden die Zellen anschließend mittels Kollagenaseverdau (108 U/mg/ml Collagenase, Worthington, USA) aus dem Gewebsverband gelöst und die freigesetzten Zellen über einen Dichtegradienten (Percoll, Pharmacia®) durch Zentrifugation separiert. Die Kardiomyozytenfraktion wurden in Zellkulturmedium (DMEM + 10% Newborne Calf Serum) resuspendiert und in einer Dichte von ca. 100.000 Kardiomyozyten pro Well auf 24 Well-Zellkulturplatten ausplattiert. Nach 24 Stunden Kulturdauer bei 37 °C und 5% CO₂ wurde dem Zellkulturmedium rekombinantes S100A1 in Konzentrationen von 1 µM bis 10 µM in zweitägigen Abständen zugefügt. Nach 7 Tagen erfolgte die Messung der Kalziumtransienten nach 1 stündiger Beladung der Zellen mit FuraPE3AM®. Die intrazellulären Ca²⁺-Transienten wurden dann mittels inverser Fluoreszenzmikroskopie (Olympus OSP 3®) sowohl unter Ruhebedingungen als auch unter elektrischer Feldstimulation (PHYWE® - Bioelektrische Meßeinheit; Frequenzen von 30-300 bpm) detektiert und quantifiziert. Fig. 2 zeigt die Originalableitungen der analysierten Kontrollzellen (2a) sowie der mit S100A1 behandelten Zellen (2b). Die statistische Auswertung zeigt, daß unter Stimulation mit S100A1 der Anstieg der Ca²⁺-Konzentration pro Zeiteinheit in der Systole (+dc/dt) im Vergleich zur Kontrollzellpopulation signifikant um das 3 fache (p<0,0002) gesteigert wird. Der Abfall der Ca²⁺-Konzentration pro Zeiteinheit in der Diastole (-dc/dt) wird unter S100A1 Stimulation im Vergleich zur Kontrollzellpopulation um das 2,2 fache (p<0,0003) beschleunigt. Gemessen wurden die Veränderungen der Ca²⁺-Konzentration pro Zeiteinheit als Quotient aus der Zeit bis zum Erreichen des Signalmaximums und der Signalamplitude (+dc/dt) bzw. als Quotient der Zeit bis zum Erreichen der Amplitudenhälfte und der Signalamplitude (-dc/dt). Mit diesem Versuchsansatz kann gezeigt werden, daß S100A1 als parakriner Faktor die Effizienz der SR Funktion verbessert.

### Beispiel 3

### Herstellung des S100A1 überexprimierenden Viruskonstruktes

Zur Herstellung des S100A1 überexprimierenden Viruskonstruktes wird folgende Strategie angewandt: Die S100A1 DNA wird hinter einem CMV-Promotor in den adenoviralen Shuttle-Vektor pAD TRACK (Tong-Chuan He et al. Proc Natl Acad Sci 95; 2509-2514) in die *multi cloning site* kloniert. Das resultierende Plasmid wird mit PME1 linearisiert und mit pEASY (Tong-Chuan He et al. Proc Natl Acad Sci 95; 2509-2514) in E.coli BJ 5183 rekombiniert. Das Rekombinationsprodukt (pAD/S100A1) entspricht einem Plasmid, welches die virale DNA des E1 und E3 deletierten Adenovirus Typ 5, die S100A1 DNA sowie eine Kanamycinresistenz enthält. Proben des Plasmids pAD/S100A1 wurden am 26. März 1999 bei der DSMZ - Deutsche Sammlung von Mikroorganismen und Zellkulturen GmbH, Mascheroder Weg 1b, 38124 Braunschweig, unter der Eingangsnummer DSM 12755 nach dem Budapester Vertrag hinterlegt. pAD/S100A1 wird dann in E.Coli DH5_{α} unter Kanamycinselektion (50 µg/ml) transformiert und vervielfältigt. pAD/S100A1 wird unter Verlust der Kanamycinresistenz mit PAC1 linearisiert und mit Hilfe von Lipofectamin (Gibco, BRL) in HEK293 Zellen zur Virusproduktion eingebracht. Nach 10 Tagen wird das Virus mit einem niedrigen Titer geerntet und HEK293 erneut infiziert. Nach mehreren Zyklen des Erntens und Reinfizierens von HEK293 Zellen liegt schließlich ein Virus mit hohem Titer vor, der vor Einbringen in den Organismus wie folgt prozessiert wird: Die HEK293 Zellen werden vor Eintreten der Zellyse abgeerntet und abzentrifugiert (3400 rpm x 10 min). Das resultierende HEK-Zellpellet wird zweimal in PBS (0.01 M , Sigma, p-3813) gewaschen und schließlich in 0.01 M TRIS-pH 8.1 aufgenommen. Dann werden die virus-enthaltenden HEK293 Zellen durch viermaliges Einfrieren (flüssiger Stickstoff) und Auftauen (bei 37 °C) lysiert und das Virus freigesetzt. HEK-Zell DNA wird durch DNAse-Verdau (bei 37 °C über 30 Min) und HEK-Zellproteine durch Freon-Extraktion aus dem Lysat entfernt. Das Virus wird mittels Ultrazentrifugation (12 h x 127000 g) über einen Caesiumchloridgradienten aufgereinigt. Die resultierende Virusfraktion wird abpunktiert und für 3 x 2 h dialysiert (1 Vol-% Sacharose in 0.01 M PBS pH 7.4) und in Aliquots bei -80 °C gelagert. Für eine gentherapeutische Behandlung werden zwischen 10⁹ bis 10¹² Viruspartikel pro Gramm Herzgewebe verwendet.

### Beispiel 4

### Gentherapie des Kaninchenherzen: Steigerung der +dP/dt und des systolischen Ejektionsdruckes infolge viraler Überexpression des Ca²⁺⁻bindenen Proteins S100A1 im Kaninchenmyokard.

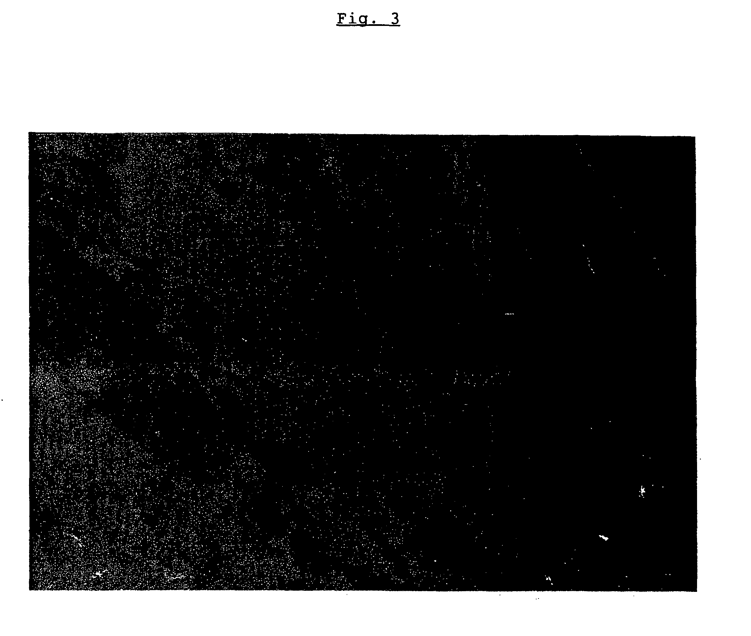

Im 4. ICR wurde durch laterale Thorakotomie der Thorax von *new zealand white rabbits* rechtsseitig eröffnet. Nach Darstellung der Aorta erfolgte die Perikarderöffnung und die Ligation der Aorta. Durch linkskavitäre Injektion wurde eine intrakoronare Perfusion mit 2 x 10¹¹ Viruspartikeln des rekombinanten S100A1 Virus (n=6), eines Virus ohne S100A1 cDNA (n=11) oder von NaCl (n=11) erreicht. 7 Tage nach der Operation wurden die Kaninchen über die A. Carotis katheterisiert. Dabei wurden die Kontraktionsgeschwindigkeit (dP/dt) und der systolische Ejektionsdruck (SEP) der Kaninchenherzen unter Basalbedingungen sowie unter Isoproterenolstimulation (0,1, 0,5 und 1,0 µg/kg/min) gemessen. Von dem in Flüssigstickstoff tiefgefrorenen Kaninchenmyokard wurden Kryoschnitte angefertigt und die Virusinfektion des Myokards durch fluoreszensmikroskopische Bestimmung der GFP Expression nachgewiesen (siehe Fig. 3).

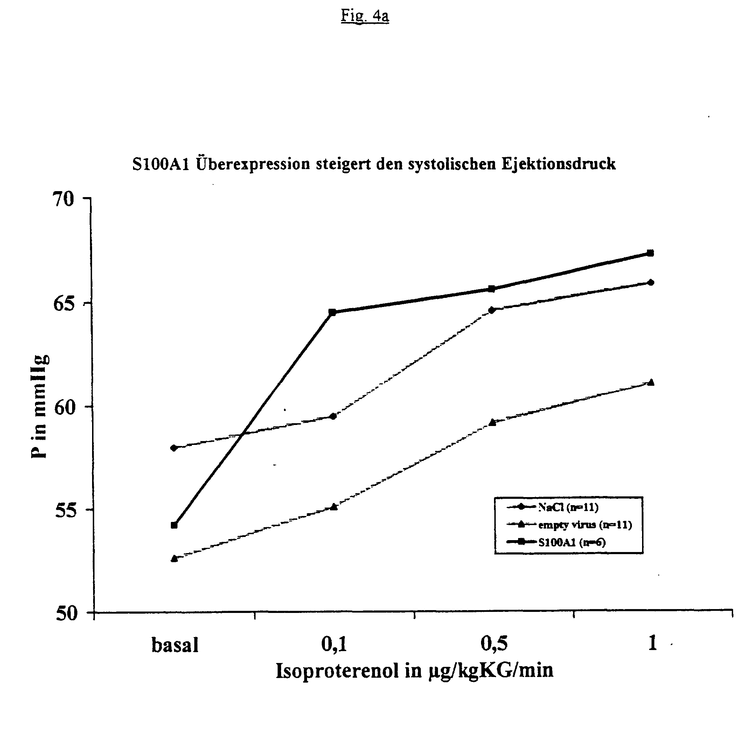

Die Injektion eines Viruskonstruktes ohne S100A1 cDNA hat eine Verminderung des systolischen Ejektionsdruckes (SEP in mmHg) der Kaninchenherzen um durchschnittlich 9% unter allen erhobenen Bedingungen im Vergleich zu den mit NaCl behandelten Tieren zur Folge. Unter Basalbedingungen verhält sich der SEP bei den mit S100A1 behandelten Tieren statistisch nicht signifikant different von beiden Kontrollgruppen. Der SEP in der Gruppe S100A1 überexprimierender Kaninchen steigt im Vergleich zu der Gruppe, die mit dem Viruskonstrukt ohne S100A1 cDNA behandelt wurden unter allen Isoproterenolstimulationen um 17% (0,1 µg/kg/min; p<0,02), 10% (0,5 µg/kg/min; p=0,06) und 11% (1,0 µg/kg/min; p<0,05) an. Unter Isoproterenolstimulation weisen die mit S100A1 behandelten Tiere einen um 4% (n.s) höheren SEP im Vergleich zu der NaCl Gruppe auf (siehe Fig. 4a).

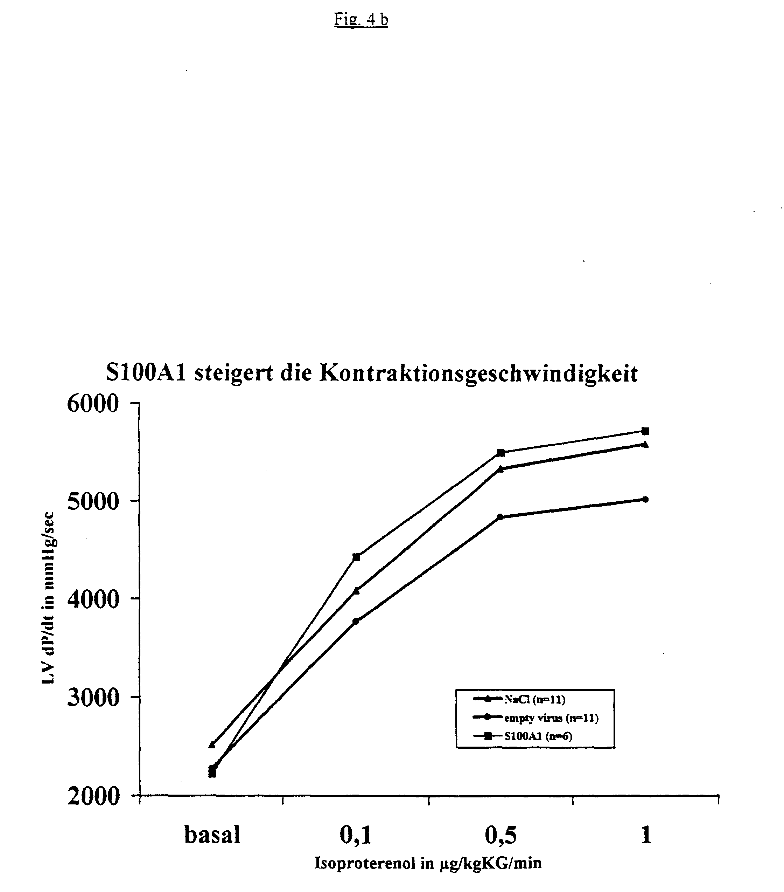

Die Kontraktionsgeschwindigkeit (dP/dt in mm Hg/s) des Herzens sinkt bei Applikation eines Viruskonstruktes ohne S100A1 cDNA im Vergleich zur NaCl Injektion unter allen gemessenen Bedingungen um durschnittlich 10 %, was wir auf eine Myokarditis zurückführen. Die S100A1 überexprimierenden Tiere zeigten unter Basalbedingungen keine statistisch abweichende dP/dt im Vergleich zu der Virus Kontrollgruppe. Demgegenüber stieg die Kontraktionskraft des Herzens in der S100A1 Gruppe unter Isoproterenolstimulation um 17 % (0,1 µg/kg/min; p<0,05), 14 % (0,5 µg/kg/min; p<0,03) und 14 % (1,0 µg/kg/min; p<0,05) im Vergleich zu der Virus Kontrolle. Die mit dem rekombinanten S100A1 Virus behandelten Tiere wiesen trotz der Myokarditis eine durchschnittlich um 5% (n.s.) erhöhte dP/dt als die NaCl Gruppe auf (siehe Fig. 4b).

### Beispiel 5

### Steigerung der Krafttransienten von skinned fibers Präparaten des Skelettmuskels der Ratte durch rekombinantes S100A1 sowie durch Peptide dieses Proteins

Die Bindung von Ca²⁺ an S100A1 führt zu einer veränderten Tertiärstruktur dieses Ca²⁺-bindenden Proteins, wobei es zu einer engen räumlichen Koordination der drei hydrophoben Proteinanteile (1. Aminosäuren 2-16 [N-terminal], vgl. SEQ ID NO: 32; 2. Aminosäuren 42-54 [hinge-region], vgl. SEQ ID NO: 34; 3. Aminosäuren 75-85 [C-terminal], vgl. SEQ ID NO: 36), kommt, die zusammen an den RyR (Ryanodinrezeptor ist ein Synonym für Ca²⁺ATPase des SR) binden, wie Daten von Treves et al (25) vermuten lassen. Das Ziel dieser Versuchsanordnung war es daher, zu untersuchen, welche funktionelle Bedeutung diese Sequenzen - in Form synthetischer Peptide - im Vergleich zum gesamten Protein für die Regulation der Ca²⁺-Freisetzung aus dem SR haben. Die Ca²⁺-Freisetzung wurde indirekt an Saponin-semipermeabilisierten Skelettmuskelfasern der Ratte über einen isometrischen Kraftgradienten gemessen. Als Kontrolle diente die Messung der isometrischen Kraft vor und nach S100A1 Peptid-/Proteinzugabe.

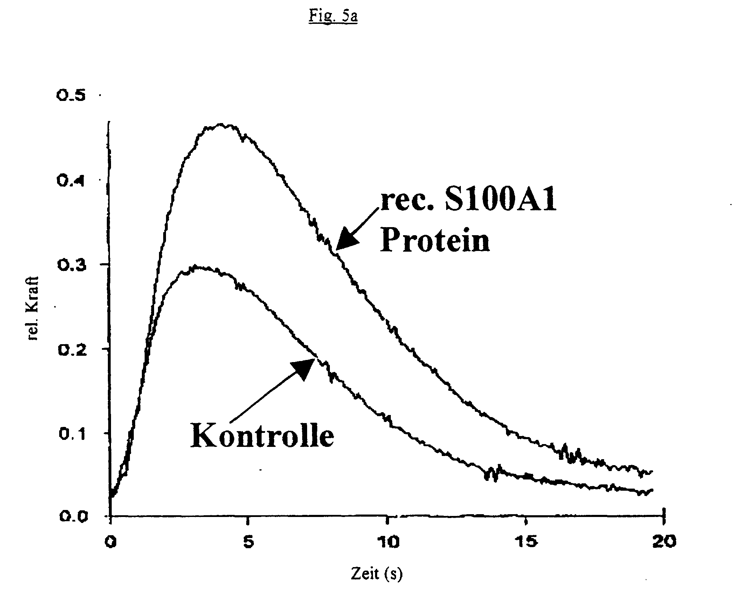

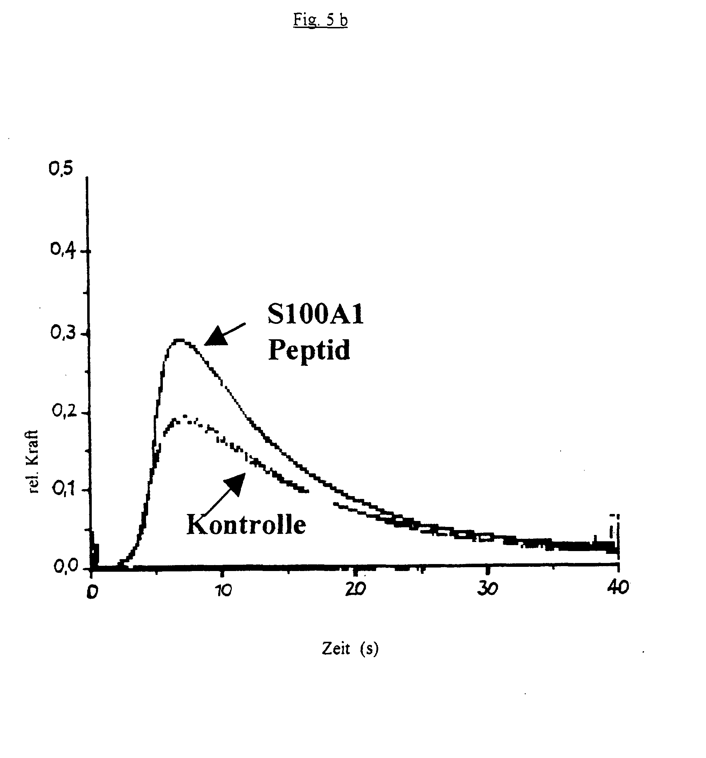

Während die einzelnen Peptide keinen Effekt zeigten, steigerte die Kombination aus "C-terminalem" Peptid und der "hinge region" die isometrische Kraftentwicklung bereits um 15%±4%. Sowohl die Kombination der drei Peptide (Fig. 5b) , als auch das rekombinante Protein (Fig. 5a) in äquimolarer Konzentration (5-10 µM) steigerte die maximale Kraftentwicklung in langsamer Skelettmuskulatur (M. soleus) gleichartig um 49%±6% bzw. 52%±7% im Vergleich zur Kontrolle bei unveränderter Ca²⁺-Sensitivität des kontraktilen Apparates.

Diese Ergebnisse zeigen, daß durch die hydrophoben Proteinanteile die Effekte von S100A1 simuliert werden können und nur durch die Kombination aller drei Peptide der volle Effekt des nativen Proteins ausgelößt wird. Damit belegen sie die Bedeutung der Ca²⁺-abhängigen koordinativen Regulation des RyR durch die hydrophoben Sequenzen von S100A1.

### Legende der Figuren

- Fig. 1:: (a) Elutionsprofil von HiTrapQ, Absorption bei 220 nm. (b) Silberfärbung nach SDS-Polyacrylamidgelelektrophorese der einzelnen Aufreinigungsstufen: 1.: Extrakt aus E. coli, 2.: von Octyl-Sepharose nicht gebundene Proteine, 3.: EGTA-Eluat von Octyl-Sepharose, 4.-5.: von HiTrapQ nicht gebundene Proteine, 6.-8.: Fraktionen des S100A1-Peaks von HiTrapQ.
- Fig. 2:: Originalableitungen der analysierten Kontrollzellen (Fig. 2a) sowie der mit S100A1 behandelten Zellen (Fig. 2b).
- Fig. 3:: Nachweis der Virusinfektion des Myokards durch fluoreszensmikroskopische Bestimmung der GFP Expression in Kryoschnitten von tiefgefrorenem Kaninchenmyokard.
- Fig. 4:: Systolischer Ejektionsdruck unter Isoproterenolstimulation bei mit S100A1 behandelten Tieren im Vergleich zur Kontroll-Gruppe (Fig. 4a). Kontraktionsgeschwindigkeit unter Isoproterenolstimulation bei mit S100A1 behandelten Tieren im Vergleich zur Kontroll-Gruppe (Fig. 4b).
- Fig. 5:: Steigerung der maximalen Kraftentwicklung in langsamer Skelettmuskulatur (M. soleus) durch Kombination der drei S100A1-Peptide (N-terminal, hinge-region, C-terminal; Fig. 5b) sowie durch rekombinantes S100A1-Protein (Fig. 5a) in äquimolarer Konzentration (5-10 µM).

- (1): Gwathmey JK, Copelas L, MacKinnon R, Schoen FJ, Feldman MD, Grossman W, Morgan JP. Abnormal intracellular calcium handling in myocardium from patients with endstage heart failure. Circ Res 1987 Jul;61(1):70-76
- (2): Beuckelmann DJ, Näbauer M, Erdmann E. Intracellular calcium handling in isolated ventricular myocytes from patients with terminal heart failure. Circulation 1992 Mar;85(3):1046-1055
- (3): Hasenfuss G, Holubarsch C, Hermann HP, Astheimer K, Pieske B, Just H. Influence of the force-frequency relationship on haemodynamics and left ventricular function in patients with non-failing hearts and in patients with dilated cardiomyopathy. Eur Heart J 1994 Feb;15(2):164-170
- (4): Bohm M, Reiger B, Schwinger RH, Erdmann E cAMP concentrations, cAMP dependent protein kinase activity, and phospholamban in non-failing and failing myocardium.Cardiovasc Res 1994 Nov;28 (11):1713-9
- (5): Packer M, Carver JR, Rodeheffer RJ, Ivanhoe RJ, DiBianco R, Zeldis SM, Hendrix GH, Bommer WJ, Elkayam U, Kukin ML, et al Effect of oral milrinone on mortality in severe chronic heart failure. The PROMISE Study Research Group. N Engl J Med 1991 Nov 21;325(21):1468-75
- (6): Cruickshank JM Phosphodiesterase III inhibitors: longterm risks and short-term benefits. Cardiovasc Drugs Ther 1993 Aug;7(4):655-60
- (7): Reddy S, Benatar D, Gheorghiade M Update on digoxin and other oral positive inotropic agents for chronic heart failure.Curr Opin Cardiol 1997 May;12(3):233-41
- (8): Zimmermann N, Boknik P, Gams E, Herzig JW, Neumann J, Scholz H Calcium sensitization as new principle of inotropic therapy in end-stage heart failure? Eur J Cardiothorac Surg 1998 Jul;14(1):70-5
- (9): Hasenfuss G, Reinecke H, Studer R, Meyer M, Pieske B, Literatur Holtz J, Holubarsch C, Posival H, Just H, Drexler H Relation between myocardial function and expression of sarcoplasmic reticulum Ca(2+)-ATPase in failing and nonfailing human myocardium. Circ Res 1994 Sep;75(3):434-442
- (10): Meyer M, Schillinger W, Pieske B, Holubarsch C, Heilmann C, Posival H, Kuwajima G, Mikoshiba K, Just H, Hasenfuss G, et al Alterations of sarcoplasmic reticulum proteins in failing human dilated cardiomyopathy Circulation 1995 Aug 15;92(4):778-784.
- (11): Studer R, Reinecke H, Bilger J, Eschenhagen T, Bohm M, Hasenfuss G, Just H, Holtz J, Drexler H. Gene expression of the cardiac Na(+)-Ca2+ exchanger in end-stage human heart failure. Circ Res 1994 Sep;75(3):443-453
- (12): Movsesian MA, Karimi M, Green K, Jones LR Ca(2+)-transporting ATPase, phospholamban, and calsequestrin levels in nonfailing and failing human myocardium. Circulation 1994 Aug;90(2):653-657
- (13): Schwinger RH, Bohm M, Schmidt U, Karczewski P, Bavendiek U, Flesch M, Krause EG, Erdmann E. Unchanged protein levels of SERCA II and phospholamban but reduced Ca2+ uptake and Ca(2+)-ATPase activity of cardiac sarcoplasmic reticulum from dilated cardiomyopathy patients compared with patients with nonfailing hearts. Circulation 1995 Dec 1;92(11):3220-3228
- (14): Arai M, Suzuki T, Nagai R. Sarcoplasmic reticulum genes are upregulated in mild cardiac hypertrophy but downregulated in severe cardiac hypertrophy induced by pressure overload. J Mol Cell Cardiol 1996 Aug;28(8):1583-1590
- (15): Schmidt U, Hajjar RJ, Helm PA, Kim CS, Doye AA, Gwathmey JK Contribution of abnormal sarcoplasmic reticulum ATPase activity to systolic and diastolic dysfunction in human heart failure. J Mol Cell Cardiol 1998 Oct; 30(10):1929-37
- (16): Schäfer BW, Heizmann CW The S100 family of EF-hand calcium-binding proteins: functions and pathology. Trends Biochem Sci 1996 Apr;21(4):134-140
- (17): Kato K, Kimura S. S100ao (alpha alpha) protein is mainly located in the heart and striated muscles. Biochim Biophys Acta 1985 Oct 17;842(2-3):146-150
- (18): Eschenhagen T et al , FASEB J. 11, 683-694; 1997
- (19): Donato R. Effect of S-100 protein on assembly of brain microtubule proteins in vitro. FEBS Lett 1983 Oct 17;162(2):310-313
- (20): Garbuglia M, Verzini M, Giambanco I, Spreca A, Donato R. Effects of calcium-binding proteins (S-100a(o), S-100a, S-100b) on desmin assembly in vitro. FASEB J 1996 Feb;10(2):317-324
- (21): Schaper J, Froede R, Hein S, Buck A, Hashizume H, Speiser B, Friedl A, Bleese N. Impairment of the myocardial ultrastructure and changes of the cytoskeleton in dilated cardiomyopathy. Circulation 1991 Feb; 83(2):504-514
- (22): Tsutsui H, Ishihara K, Cooper G 4^{th} Cytoskeletal role in the contractile dysfunction of hypertrophied myocardium. Science 1993 Apr 30;260(5108):682-687
- (23): Baudier J, Bergeret E, Bertacchi N, Weintraub H, Gagnon J, Garin J Interactions of myogenic bHLH transcription factors with calcium-binding calmodulin and S100a (alpha alpha) proteins. Biochemistry 1995 Jun 20; 34(24):7834-7846
- (24): Wakasaki H, Koya D, Schoen FJ, Jirousek MR, Ways DK, Hoit BD, Walsh RA, King GL Targeted overexpression of protein kinase C beta2 isoform in myocardium causes cardiomyopathy.Proc Natl Acad Sci U S A 1997 Aug 19;94 (17):9320-5
- (25): Treves S, Scutari E, Robert M, Groh S, Ottolia M, Prestipino G, Ronjat M, Zorzato F Interaction of S100A1 with the Ca2+ release channel (ryanodine receptor) of skeletal muscle. Biochemistry 1997 Sep 23;36(38):11496-11503

## Patentansprüche

1. Arzneimittel zur Behandlung von Herzerkrankungen aus der Gruppe bestehend aus primären Kardiomyopathien und sekundären Kardiomyopathien, **dadurch gekennzeichnet, daß** es für ein oder mehrere S100-Proteine aus der Gruppe bestehend aus S100A1, S100A2, S100A3, S100A4, S100A5, S100A6, S100A7, S100A8, S100A9, S100A10, S100A11, S100A12 und S100A13, für eine oder mehrere Mutanten derselben mit einer Homologie von mindestens 60% dazu, die die systolische Ca²⁺-Freisetzung aus dem sarkoplasmatischen Retikulum erhöhen und eine Ca²⁺-Wiederaufnahme in das sarkoplasmatische Retikulum beschleunigen, oder für ein oder mehrere hydrophobe Peptide aus der Gruppe bestehend aus Peptiden gemäß SEQ ID NO: 36 und 39 kodierende Nukleinsäuresequenz(en) enthält, wobei die Nukleinsäuresequenz(en) gegebenenfalls mit pharmazeutisch verträglichen Hilfsund/oder Trägerstoffen formuliert ist (sind).

2. Arzneimittel zur Behandlung von Herzerkrankungen aus der Gruppe bestehend aus primären Kardiomyopathien und sekundären Kardiomyopathien, **dadurch gekennzeichnet, daß** es einen Gentransfervektor enthält, der für ein oder mehrere S100-Proteine aus der Gruppe bestehend aus S100A1, S100A2, S100A3, S100A4, S100A5, S100A6, S100A7, S100A8, S100A9, S100A10, S100A11, S100A12 und S100A13, für eine oder mehrere Mutanten derselben mit einer Homologie von mindestens 60% dazu, die die systolische Ca²⁺-Freisetzung aus dem sarkoplasmatischen Retikulum erhöhen und eine Ca²⁺-Wiederaufnahme in das sarkoplasmatische Retikulum beschleunigen, oder für ein oder mehrere hydrophobe Peptide aus der Gruppe bestehend aus Peptiden gemäß SEQ ID NO: 36 und 39 kodierende Nukleinsäuresequenz(en) enthält, wobei der Vektor gegebenenfalls mit pharmazeutisch verträglichen Hilfs- und/oder Trägerstoffen formuliert ist.

3. Arzneimittel nach Anspruch 1 oder 2, **dadurch gekennzeichnet, daß** die primären Kardiomyopathien familiär vererbte Kardiomyopathien und Kardiomyopathien auf dem Boden von Spontanmutationen umfassen.

4. Arzneimittel nach Anspruch 1 oder 2, **dadurch gekennzeichnet, daß** die sekundären Kardiomyopathien ischämische Kardiomyopathie auf dem Boden einer Arteriosklerose, dilatative Kardiomyopathie auf dem Boden einer infektiösen oder toxischen Erkrankung des Myokards, hypertensive Herzerkrankung auf dem Boden einer pulmonalarteriellen und/oder einer arteriellen Hypertonie, strukturelle Herzerkrankungen auf dem Boden von Rhythmusstörungen und Erkrankungen der Herzklappen umfassen.

5. Arzneimittel zur Behandlung der Herzinsuffizienz, **dadurch gekennzeichnet, daß** es für ein oder mehrere S100-Proteine aus der Gruppe bestehend aus S100A1, S100A2, S100A3, S100A4, S100A5, S100A6, S100A7, S100A8, S100A9, S100A10, S100A11, S100A12 und S100A13, für eine oder mehrere Mutanten derselben mit einer Homologie von mindestens 60% dazu, die die systolische Ca²⁺-Freisetzung aus dem sarkoplasmatischen Retikulum erhöhen und eine Ca²⁺-Wiederaufnahme in das sarkoplasmatische Retikulum beschleunigen, oder ein oder mehrere hydrophobe Peptide aus der Gruppe bestehend aus Peptiden gemäß SEQ ID NO: 36 und 39 kodierende Nukleinsäuresequenz(en) enthält, wobei die Nukleinsäuresequenz(en) gegebenenfalls mit pharmazeutisch verträglichen Hilfs- und/oder Trägerstoffen formuliert ist (sind).

6. Arzneimittel zur Behandlung der Herzinsuffizienz, **dadurch gekennzeichnet, daß** es einen Gentransfervektor enthält, der für ein oder mehrere S100-Proteine aus der Gruppe bestehend aus S100A1, S100A2, S100A3, S100A4, S100A5, S100A6, S100A7, S100A8, S100A9, S100A10, S100A11, S100A12 und S100A13, für ein oder mehrere Mutanten derselben mit einer Homologie von Mindestens 60% dazu, die die systolische Ca²⁺-Freisetzung aus dem sarkoplasmatischen Retikulum erhöhen und eine Ca²⁺-Wiederaufnahme in das sarkoplasmatische Retikulum beschleunigen, oder ein oder mehrere hydrophobe Peptide aus der Gruppe bestehend aus Peptiden gemäß SEQ ID NO: 36 und 39 kodierende Nukleinsäuresequenz(en) enthält, wobei der Vektor gegebenenfalls mit pharmazeutisch verträglichen Hilfs- und/oder Trägerstoffen formuliert ist.

7. Arzneimittel nach den Ansprüchen 1 bis 6, **dadurch gekennzeichnet, daß** S100A1 die in SEQ ID NO: 2 gezeigte Aminosäuresequenz aufweist.

8. Arzneimittel nach den Ansprüchen 1 bis 6, **dadurch gekennzeichnet, daß** das Peptid die in SEQ ID NO: 36 gezeigte Aminosäuresequenz aufweist.

9. Arzneimittel nach den Ansprüchen 1 bis 6, **dadurch gekennzeichnet, daß** es eine Kombination von mindestens zwei der in SEQ ID NO: 32, 34, 36, 37, 38 und 39 dargestellten Peptide enthält.

10. Arzneimittel nach den Ansprüchen 1 bis 6, **dadurch gekennzeichnet, daß** es eine für S100A1 kodierende Nukleinsäuresequenz gemäß SEQ ID NO: 1 oder einen diese Sequenz enthaltenden Gentransfervektor enthält.

11. Arzneimittel nach den Ansprüchen 1 bis 6, **dadurch gekennzeichnet, daß** die für die Peptide kodierenden Nukleinsäuresequenzen aus der Gruppe bestehend aus SEQ ID NO: 35 und aus den für die Peptide gemäß SEQ ID NO: 39 kodierenden Nukleinsäuresequenzen ausgewählt sind oder in Form von diese Sequenzen enthaltenden Gentransfervektor(en) enthalten sind.

12. Arzneimittel nach den Ansprüchen 1 bis 6, **dadurch gekennzeichnet, daß** es eine Kombination von mindestens zwei der für SEQ ID NO: 32, 34, 36, 37, 38 und 39 kodierenden Nukleinsäuresequenzen, vorzugsweise mindestens zwei der in SEQ ID NO: 31, 33, 35 dargestellten Nukleinsäuresequenzen und für SEQ ID NO: 37, 38 und 39 kodierende Sequenzen, oder einen oder mehrere diese Kombination enthaltenden Gentransfervektor enthält.

13. Arzneimittel nach den Ansprüchen 1 bis 12, **dadurch gekennzeichnet, daß** es zur oralen, intravenösen, intraarteriellen oder intrakoronaren Applikation formuliert ist.

14. Verwendung eines oder mehrerer S100-Proteine, einer oder mehrerer Mutanten derselben, die die systolische Ca²⁺-Freisetzung aus dem sarkoplasmatischen Retikulum erhöhen und eine Ca²⁺-Wiederaufnahme in das sarkoplasmatische Retikulum beschleunigen, oder eines oder mehrerer hydrophober Peptide aus der Gruppe bestehend aus Peptiden gemäß SEQ ID NO: 36 und 39 zur Herstellung eines Arzneimittels zur Behandlung von Herzerkrankungen aus der Gruppe bestehend aus primären Kardiomyopathien und sekundären Kardiomyopathien.

15. Verwendung einer oder mehrerer Nukleinsäuresequenzen, die für ein oder mehrere S100-Proteine, eine oder mehrere Mutanten derselben, die die systolische Ca²⁺-Freisetzung aus dem sarkoplasmatischen Retikulum erhöhen und eine Ca²⁺-Wiederaufnahme in das sarkoplasmatische Retikulum beschleunigen, oder ein oder mehrerer hydrophober Peptide aus der Gruppe bestehend aus Peptiden gemäß SEQ ID NO: 36 und 39 kodieren, zur Herstellung eines pharmazeutischen Präparats zur Behandlung von Herzerkrankungen aus der Gruppe bestehend aus primären Kardiomyopathien und sekundären Kardiomyopathien, wobei die Nukleinsäure(n) gegebenenfalls mit pharmazeutisch verträglichen Hilfs- und/oder Trägerstoffen formuliert ist (sind).

16. Verwendung eines Gentransfervektors, der für ein oder mehrere S100-Proteine, eine oder mehrere Mutanten derselben, die die systolische Ca²⁺-Freisetzung aus dem sarkoplasmatischen Retikulum erhöhen und eine Ca²⁺-Wiederaufnahme in das sarkoplasmatische Retikulum beschleunigen, oder ein oder mehrere hydrophobe Peptide aus der Gruppe bestehend aus Peptiden gemäß SEQ ID NO: 36 und 39 kodierende Nukleinsäuresequenz(en) enthält, zur Herstellung eines pharmazeutischen Präparats zur Behandlung von Herzerkrankungen aus der Gruppe bestehend aus primären Kardiomyopathien und sekundären Kardiomyopathien, wobei der Vektor gegebenenfalls mit pharmazeutisch verträglichen Hilfs- und/oder Trägerstoffen formuliert ist.

17. Verwendung nach den Ansprüchen 14 bis 16, **dadurch gekennzeichnet, daß** die primären Kardiomyopathien familiär vererbte Kardiomyopathien und Kardiomyopathien auf dem Boden von Spontanmutationen umfassen.

18. Verwendung nach den Ansprüchen 14 bis 16, **dadurch gekennzeichnet, daß** die sekundären Kardiomyopathien ischämische Kardiomyopathie auf dem Boden einer Arteriosklerose, dilatative Kardiomyopathie auf dem Boden einer infektiösen oder toxischen Erkrankung des Myokards, hypertensive Herzerkrankung auf dem Boden einer pulmonalarteriellen und/oder einer arteriellen Hypertonie, strukturelle Herzerkrankungen auf dem Boden von Rhythmusstörungen und Erkrankungen der Herzklappen umfaßt.

19. Verwendung eines oder mehrerer S100-Proteine, einer oder mehrere Mutanten derselben, die die systolische Ca²⁺-Freisetzung aus dem sarkoplasmatischen Retikulum erhöhen und eine Ca²⁺-Wiederaufnahme in das sarkoplasmatische Retikulum beschleunigen, oder eines oder mehrerer hydrophober Peptide aus der Gruppe bestehend aus Peptiden gemäß SEQ ID NO: 36 und 39 zur Herstellung eines pharmazeutischen Präparats zur Behandlung der Herzinsuffizienz.

20. Verwendung einer oder mehrerer Nukleinsäuresequenzen, die für ein oder mehrere S100-Proteine, eine oder mehrere Mutanten derselben, die die systolische Ca²⁺-Freisetzung aus dem sarkoplasmatischen Retikulum erhöhen und eine Ca²⁺-Wiederaufnahme in das sarkoplasmatische Retikulum beschleunigen, oder ein oder mehrere hydrophobe Peptide aus der Gruppe bestehend aus Peptiden gemäß SEQ ID NO: 36 und 39 kodieren, zur Herstellung eines pharmazeutischen Präparats zur Behandlung der Herzinsuffizienz, wobei die Nukleinsäure(n) gegebenenfalls mit pharmazeutisch verträglichen Hilfs- und/oder Trägerstoffen formuliert ist (sind).

21. Verwendung eines Gentransfervektors, der für ein oder mehrere S100-Proteine, für eine oder mehrere Mutanten derselben, die die systolische Ca²⁺-Freisetzung aus dem sarkoplasmatischen Retikulum erhöhen und eine Ca²⁺-Wiederaufnahme in das sarkoplasmatische Retikulum beschleunigen, oder für ein oder mehrere hydrophobe Peptide aus der Gruppe bestehend aus Peptiden gemäß SEQ ID NO: 36 und 39 kodierende Nukleinsäuresequenz(en) enthält, zur Herstellung eines pharmazeutischen Präparats zur Behandlung der Herzinsuffizienz, wobei der Vektor gegebenenfalls mit pharmazeutisch verträglichen Hilfs- und/oder Trägerstoffen formuliert ist.

22. Verwendung nach den Ansprüchen 19 bis 21, **dadurch gekennzeichnet, daß** das S100-Protein aus der Gruppe bestehend aus S100β, S100A1, S100A2, S100A4 und S100A6 ausgewählt ist.

23. Verwendung nach Anspruch 22, **dadurch gekennzeichnet, daß** S100A1 die in SEQ ID NO: 2 gezeigte Aminosäuresequenz aufweist.

24. Verwendung nach Anspruch 21, **dadurch gekennzeichnet, daß** das Peptide die in SEQ ID NO: 36 gezeigte Aminosäuresequenz aufweist .

25. Verwendung nach den Ansprüchen 14 bis 22, **dadurch gekennzeichnet, daß** man eine Kombination von mindestens zwei der in SEQ ID NO: 32, 34, 36, 37, 38 und 39 dargestellten Peptide verwendet.

26. Verwendung nach den Ansprüchen 15 bis 22, **dadurch gekennzeichnet, daß** man eine für S100A1 kodierende Nukleinsäuresequenz gemäß SEQ ID NO: 1 oder einen diese Sequenz enthaltenden Gentransfervektor verwendet.

27. Verwendung nach den Ansprüchen 16 bis 22, **dadurch gekennzeichnet, daß** die für die Peptide kodierenden Nukleinsäuresequenzen aus SEQ ID NO: 35 oder diese Sequenz enthaltenden Gentransfervektoren ausgewählt sind.

28. Verwendung nach den Ansprüchen 15 bis 22, **dadurch gekennzeichnet, daß** man eine Kombination von mindestens zwei der für SEQ ID NO: 32, 34, 36, 37, 38 und 39 kodierenden Nukleinsäuresequenzen, vorzugsweise mindestens zwei der in SEQ ID NO: 31, 33, 35 dargestellten Nukleinsäuresequenzen und für SEQ ID NO: 37, 38 und 39 kodierende Sequenzen, oder einen oder mehrere diese Kombination umfassenden Gentransfervektoren verwendet.

29. Verwendung nach den Ansprüchen 14 bis 28, **dadurch gekennzeichnet, daß** das Arzneimittel zur oralen, intravenösen, intraarteriellen oder intrakoronaren Applikation formuliert ist.

30. Peptid, **dadurch gekennzeichnet, daß** es aus der in SEQ ID NO: 34, 36, 38 oder 39 dargestellten Sequenz besteht.

31. Nukleinsäure, **dadurch gekennzeichnet, daß** sie für die Peptide nach Anspruch 30 kodiert.

32. Gentransfervektor gemäß DSM 12755.

33. Verfahren zur Herstellung eines für ein oder mehrere S100-Proteine aus der Gruppe bestehend aus S140A1, S100A2, S100A3, S100A4, S100A5, S100A6, S100A7, S100A8, S100A9, S100A10, S100A11, S100A12 und S100A13, für eine oder mehrere Mutanten derselben mit einer Homologie von mindestens 60% dazu, die die systolische Ca²⁺-Freisetzung aus dem sarkoplasmatischen Retikulum erhöhen und eine Ca²⁺-Wiederaufnahme in das sarkoplasmatische Retikulum beschleunigen, oder für ein oder mehrere hydrophobe Peptide aus der Gruppe bestehend aus Peptiden gemäß SEQ ID NO: 36 und 39 kodierenden Gentransfervektors, **dadurch gekennzeichnet, daß** man eine für das (die) Protein(e), die Mutante(n) oder die Peptide kodierende(n) Nukleinsäuresequenz(en) in einen oder mehrere zur Gentherapie geeignete Vektoren kloniert.

34. Verfahren nach Anspruch 33, **dadurch gekennzeichnet, daß** man die kodierende (n) Nukleinsäuresequenz (en) aus der Gruppe der in SEQ ID NO: 1 bis 26 dargestellten Nukleinsäuresequenzen auswählt.

35. Verfahren zur Herstellung des Gentransfervektors nach Anspruch 32, **dadurch gekennzeichnet, daß** man die in SEQ ID NO: 1 dargestellte cDNA in den multi cloning site des Shuttle-Vektors pAD TRACK kloniert, mit PME1 linearisiert und mit pEASY in *E. coli* BJ 5183 rekombiniert.

## Claims

1. Medicament for the treatment of cardiac disorders from the group consisting of primary cardiomyopathies and secondary cardiomyopathies, **characterized in that** it comprises nucleic acid sequence(s) coding for one or more S100 proteins from the group consisting of S100A1, S100A2, S100A3, S100A4, S100A5, S100A6, S100A7, S100A8, S100A9, S100A10, S100A11, S100A12 and S100A13, for one or more mutants thereof having a homology of at least 60% thereto, which increase the systolic Ca²⁺ release from the sarcoplasmic reticulum and increase the rate of Ca²⁺ uptake into the sarcoplasmic reticulum, or for one or more hydrophobic peptides from the group consisting of peptides as shown in SEQ ID NO: 36 and 39, where the nucleic acid sequence(s) is(are) formulated where appropriate with pharmaceutically acceptable excipients and/or carriers.

2. Medicament for the treatment of cardiac disorders from the group consisting of primary cardiomyopathies and secondary cardiomyopathies, **characterized in that** it comprises a gene transfer vector coding for one or more S100 proteins from the group consisting of S100A1, S100A2, S100A3, S100A4, S100A5, S100A6, S100A7, S100A8, S100A9, S100A10, S100A11, S100A12 and S100A13, for one or more mutants thereof having a homology of at least 60% thereto, which increase the systolic Ca²⁺ release from the sarcoplasmic reticulum and increase the rate of Ca²⁺ uptake into the sarcoplasmic reticulum, or for one or more hydrophobic peptides from the group consisting of peptides as shown in SEQ ID NO: 36 and 39, where the vector is formulated where appropriate with pharmaceutically acceptable excipients and/or carriers.

3. Medicament according to Claim 1 or 2, **characterized in that** the primary cardiomyopathies include familial cardiomyopathies and cardiomyopathies based on spontaneous mutations.

4. Medicament according to Claim 1 or 2, **characterized in that** the secondary cardiomyopathies include ischaemic cardiomyopathy based on an arteriosclerosis, dilated cardiomyopathy based on an infectious or toxic disorder of the myocardium, hypertensive heart disease based on a pulmonary and/or an essential hypertension, structural heart diseases based on rhythm disturbances and disorders of the heart valves.

5. Medicament for the treatment of heart failure, **characterized in that** it comprises nucleic acid sequence(s) coding for one or more S100 proteins from the group consisting of S100A1, S100A2, S100A3, S100A4, S100A5, S100A6, S100A7, S100A8, S100A9, S100A10, S100A11, S100A12 and S100A13, for one or more mutants thereof having a homology of at least 60% thereto, which increase the systolic Ca²⁺ release from the sarcoplasmic reticulum and increase the rate of Ca²⁺ uptake into the sarcoplasmic reticulum, or for one or more hydrophobic peptides from the group consisting of peptides as shown in SEQ ID NO: 36 and 39, where the nucleic acid sequence(s) is(are) formulated where appropriate with pharmaceutically acceptable excipients and/or carriers.

6. Medicament for the treatment of heart failure, **characterized in that** it comprises a gene transfer vector coding for one or more S100 proteins from the group consisting of S100A1, S100A2, S100A3, S100A4, S100A5, S100A6, S100A7, S100A8, S100A9, S100A10, S100A11, S100A12 and S100A13, for one or more mutants thereof having a homology of at least 60% thereto, which increase the systolic Ca²⁺ release from the sarcoplasmic reticulum and increase the rate of Ca²⁺ uptake into the sarcoplasmic reticulum, or for one or more hydrophobic peptides from the group consisting of peptides as shown in SEQ ID NO: 36 and 39, where the vector is formulated where appropriate with pharmaceutically acceptable excipients and/or carriers.

7. Medicament according to Claims 1 to 6, **characterized in that** S100A1 has the amino acid sequence shown in SEQ ID NO: 2.

8. Medicament according to Claims 1 to 6, **characterized in that** the peptide has the amino acid sequence shown in SEQ ID NO: 36.

9. Medicament according to Claims 1 to 6, **characterized in that** it comprises a combination of at least two of the peptides represented in SEQ ID NO: 32, 34, 36, 37, 38 and 39.

10. Medicament according to Claims 1 to 6, **characterized in that** it comprises a nucleic acid sequence coding for S100A1 as shown in SEQ ID NO: 1 or a gene transfer vector comprising this sequence.

11. Medicament according to Claims 1 to 6, **characterized in that** the nucleic acid sequences coding for the peptides are selected from the group consisting of SEQ ID NO: 35 and from the nucleic acid sequences coding for the peptides as shown in SEQ ID NO: 39, or are present in the form of gene transfer vector(s) comprising these sequences.

12. Medicament according to Claims 1 to 6, **characterized in that** it comprises a combination of at least two of the nucleic acid sequences coding for SEQ ID NO: 32, 34, 36, 37, 38 and 39, preferably at least two of the nucleic acid sequences represented in SEQ ID NO: 31, 33, 35, and sequences coding for SEQ ID NO: 37, 38 and 39, or one or more gene transfer vector comprising this combination.

13. Medicament according to Claims 1 to 12, **characterized in that** it is formulated for oral, intravenous, intraarterial or intracoronary administration.

14. Use of one or more S100 proteins, of one or more mutants thereof which increase the systolic Ca²⁺ release from the sarcoplasmic reticulum and increase the rate of Ca²⁺ uptake into the sarcoplasmic reticulum, or of one or more hydrophobic peptides from the group consisting of peptides as shown in SEQ ID NO: 36 and 39 for producing a medicament for the treatment of cardiac disorders from the group consisting of primary cardiomyopathies and secondary cardiomyopathies.

15. Use of one or more nucleic acid sequences which code for one or more S100 proteins, one or more mutants thereof which increase the systolic Ca²⁺ release from the sarcoplasmic reticulum and increase the rate of Ca²⁺ uptake into the sarcoplasmic reticulum, or one or more hydrophobic peptides from the group consisting of peptides as shown in SEQ ID NO: 36 and 39, for producing a pharmaceutical product for the treatment of cardiac disorders from the group consisting of primary cardiomyopathies and secondary cardiomyopathies, where the nucleic acid(s) is(are) formulated where appropriate with pharmaceutically acceptable excipients and/or carriers.

16. Use of a gene transfer vector which comprises nucleic acid sequence(s) coding for one or more S100 proteins, one or more mutants thereof which increase the systolic Ca²⁺ release from the sarcoplasmic reticulum and increase the rate of Ca²⁺ uptake into the sarcoplasmic reticulum, or one or more hydrophobic peptides from the group consisting of peptides as shown in SEQ ID NO: 36 and 39, for producing a pharmaceutical product for the treatment of cardiac disorders from the group consisting of primary cardiomyopathies and secondary cardiomyopathies, where the vector is formulated where appropriate with pharmaceutically acceptable excipients and/or carriers.

17. Use according to Claims 14 to 16, **characterized in that** the primary cardiomyopathies include familial cardiomyopathies and cardiomyopathies based on spontaneous mutations.

18. Use according to Claims 14 to 16, **characterized in that** the secondary cardiomyopathies include ischaemic cardiomyopathy based on an arteriosclerosis, dilated cardiomyopathy based on an infectious or toxic disorder of the myocardium, hypertensive heart disease based on a pulmonary and/or an essential hypertension, structural heart diseases based on rhythm disturbances and disorders of the heart valves.

19. Use of one or more S100 proteins, of one or more mutants thereof which increase the systolic Ca²⁺ release from the sarcoplasmic reticulum and increase the rate of Ca²⁺ uptake into the sarcoplasmic reticulum, or of one or more hydrophobic peptides from the group consisting of peptides as shown in SEQ ID NO: 36 and 39 for producing a pharmaceutical product for the treatment of heart failure.

20. Use of one or more nucleic acid sequences which code for one or more S100 proteins, one or more mutants thereof which increase the systolic Ca²⁺ release from the sarcoplasmic reticulum and increase the rate of Ca²⁺ uptake into the sarcoplasmic reticulum, or one or more hydrophobic peptides from the group consisting of peptides as shown in SEQ ID NO: 36 and 39, for producing a pharmaceutical product for the treatment of heart failure, where the nucleic acid(s) is(are) formulated where appropriate with pharmaceutically acceptable excipients and/or carriers.

21. Use of a gene transfer vector which comprises nucleic acid sequence(s) coding for one or more S100 proteins, one or more mutants thereof which increase the systolic Ca²⁺ release from the sarcoplasmic reticulum and increase the rate of Ca²⁺ uptake into the sarcoplasmic reticulum, or one or more hydrophobic peptides from the group consisting of peptides as shown in SEQ ID NO: 36 and 39, for producing a pharmaceutical product for the treatment of heart failure, where the vector is formulated where appropriate with pharmaceutically excipients and/or carriers.

22. Use according to Claims 19 to 21, **characterized in that** the S100 protein is selected from the group consisting of S100β, S100A1, S100A2, S100A4 and S100A6.

23. Use according to Claim 22, **characterized in that** S100A1 has the amino acid sequence shown in SEQ ID NO: 2.

24. Use according to Claim 21, **characterized in that** the peptide has the amino acid sequence shown in SEQ ID NO: 36.

25. Use according to Claims 14 to 22, **characterized in that** a combination of at least two of the peptides represented in SEQ ID NO: 32, 34, 36, 37, 38 and 39 is used.

26. Use according to Claims 15 to 22, **characterized in that** a nucleic acid sequence coding for S100A1 as shown in SEQ ID NO: 1 or a gene transfer vector comprising this sequence is used.

27. Use according to Claims 16 to 22, **characterized in that** the nucleic acid sequences coding for the peptides are selected from SEQ ID NO: 35 or gene transfer vectors comprising this sequence.

28. Use according to Claims 15 to 22, **characterized in that** it comprises a combination of at least two of the nucleic acid sequences coding for SEQ ID NO: 32, 34, 36, 37, 38 and 39, preferably at least two of the nucleic acid sequences represented in SEQ ID NO: 31, 33, 35, and sequences coding for SEQ ID NO: 37, 38 and 39, or one or more gene transfer vectors comprising this combination.

29. Use according to Claims 14 to 28, **characterized in that** the medicament is formulated for oral, intravenous, intraarterial or intracoronary administration.

30. Peptide, **characterized in that** it consists of the sequence represented in SEQ ID NO: 34, 36, 38 or 39.

31. Nucleic acid, **characterized in that** it codes for the peptides according to Claim 30.

32. Gene transfer vector according to DSM 12755.

33. Process for producing a gene transfer vector coding for one or more S100 proteins from the group consisting of S100A1, S100A2, S100A3, S100A4, S100A5, S100A6, S100A7, S100A8, S100A9, S100A10, S100A11, S100A12 and S100A13, for one or more mutants thereof having a homology of at least 60% thereto, which increase the systolic Ca²⁺ release from the sarcoplasmic reticulum and increase the rate of Ca²⁺ uptake into the sarcoplasmic reticulum, or for one or more hydrophobic peptides from the group consisting of peptides as shown in SEQ ID NO: 36 and 39, **characterized in that** a nucleic acid sequence(s) coding for the protein(s), the mutant(s) or the peptides is cloned into one or more vectors suitable for gene therapy.

34. Process according to Claim 33, **characterized in that** the coding nucleic acid sequence(s) is(are) selected from the group of nucleic acid sequences represented in SEQ ID NO: 1 to 26.

35. Process for producing the gene transfer vector according to Claim 32, **characterized in that** the cDNA represented in SEQ ID NO: 1 is cloned into the multi cloning site of the shuttle vector pAD TRACK, linearized with PME1 and combined with pEASY in *E.coli* BJ 5183.

## Revendications

1. Médicament traitant les affections cardiaques du groupe constitué par les cardiomyopathies primaires et les cardiomyopathies secondaires, **caractérisé par le fait qu'**il contient une séquence (des séquences) d'acides nucléiques codant pour une ou plusieurs protéines S100 du groupe constitué par S100A1, S100A2, S100A3, S100A4, S100A5, S100A6, S100A7, S100A8, S100A9, S100A10, S100A11, S100A12, S100A13, pour un ou plusieurs de leurs mutants, présentant une homologie d'au moins 60 %, qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO: 36 et 39, la séquence (les séquences) d'acides nucléiques est (sont) le cas échéant formulé(es) avec des excipients ou des supports pharmaceutiquement acceptables.

2. Médicament traitant les affections cardiaques du groupe constitué par les cardiomyopathies primaires et les cardiomyopathies secondaires, **caractérisé en ce qu'**il contient un vecteur de transfert génétique qui contient une séquence (des séquences) d'acides nucléiques codant pour une ou plusieurs protéines S100 du groupe constitué par S100A1, S100A2, S100A3, S100A4, S100A5, S100A6, S100A7, S100A8, S100A9, S100A10, S100A11, S100A12, S100A13, pour un ou plusieurs de ses mutants, présentant une homologie d'au moins 60 %, qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39, le vecteur étant le cas échéant formulé avec des excipients ou des supports pharmaceutiquement acceptables.

3. Médicament selon la revendication 1 ou 2, **caractérisé en ce que** les cardiomyopathies primaires englobent des cardiomyopathies héréditaires et des cardiomyopathies sur terrain de mutations spontanées.

4. Médicament selon la revendication 1 ou 2, **caractérisé en ce que** les cardiomyopathies secondaires englobent la cardiomyopathie ischémique sur un terrain d'artériosclérose, de cardiomyopathie dilatative sur un terrain d'affection infectieuse ou toxique du myocarde, l'affection cardiaque hypertensive sur terrain d'hypertonie des artères pulmonaires et/ou hypertonie artérielle, des affections cardiaques structurelles sur terrains de troubles du rythme et d'affections des valves du coeur.

5. Médicament traitant l'insuffisance cardiaque, **caractérisé en ce qu'**il contient une ou des séquences d'acides nucléiques codant pour une ou plusieurs protéines S100 du groupe constitué par S100A1, S100A2, S100A3, S100A4, S100A5, S100A6, S100A7, S100A8, S100A9, S100A10, S100A11, S100A12, S100A13, pour un ou plusieurs de ses mutants, présentant une homologie d'au moins 60 %, qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39, la séquence (les séquences) d'acides nucléiques étant le cas échéant formulée avec des excipients ou des supports pharmaceutiquement acceptables.

6. Médicament traitant l'insuffisance cardiaque, **caractérisé en ce qu'**il contient un vecteur de transfert génétique qui contient une ou des séquences d'acides nucléiques codant pour une ou plusieurs protéines S100 du groupe constitué par S100A1, S100A2, S100A3, S100A4, S100A5, S100A6, S100A7, S100A8, S100A9, S100A10, S100A11, S100A12, S100A13, pour un ou plusieurs de ses mutants, présentant une homologie d'au moins 60 %, qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39, le vecteur étant le cas échéant formulé avec des excipients ou des supports pharmaceutiquement acceptables.

7. Médicament selon les revendications 1 à 6, **caractérisé en ce que** S100A1 présente la séquence d'acides aminés apparaissant dans SEQ ID NO : 2.

8. Médicament selon les revendications 1 à 6, **caractérisé en ce que** le peptide présente la séquence d'acides aminés apparaissant dans SEQ ID NO : 36.

9. Médicament selon les revendications 1 à 6, **caractérisé en ce qu'**il contient une combinaison d'au moins deux peptides présents dans SEQ ID NO : 32, 34, 36, 37, 38 et 39.

10. Médicament selon les revendications 1 à 6, **caractérisé en ce qu'**il contient une séquence d'acides nucléiques codant pour S100A1 selon SEQ ID NO : 1 ou un vecteur de transfert génétique contenant cette séquence.

11. Médicament selon les revendications 1 à 6, **caractérisé en ce que** les séquences d'acides nucléiques codant pour les peptides sont choisies dans le groupe formé par SEQ ID NO : 35 et des séquences d'acides nucléiques codant pour les peptides selon SEQ ID NO : 39 ou qu'elles sont contenues sous la forme d'un (ou de plusieurs) vecteur(s) de transfert génétique.

12. Médicament selon les revendications 1 à 6, **caractérisé en ce qu'**il contient une combinaison d'au moins deux des séquences d'acides nucléiques codant pour SEQ ID NO : 32, 34, 36, 37, 38 et 39, de préférence au moins deux des séquences d'acides nucléiques présentes parmi SEQ ID NO : 31, 33, 35 et des séquences codant pour SEQ ID NO : 37, 38 et 39, ou un ou plusieurs vecteurs de transfert génétique contenant cette combinaison.

13. Médicament selon les revendications 1 à 12, **caractérisé en ce qu'**il est formulé pour une application orale, intraveineuse, intra-artérielle ou intra-coronaire.

14. Utilisation d'une ou plusieurs protéines S100 ou de plusieurs de leurs mutants qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39 pour fabriquer un médicament visant à traiter les affections cardiaques du groupe formé par les cardiomyopathies primaires et les cardiomyopathies secondaires.

15. Utilisation d'une ou plusieurs séquences d'acides nucléiques qui codent pour une ou plusieurs protéines S 100, un ou plusieurs de leurs mutants qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39 pour fabriquer une préparation pharmaceutique visant à traiter les affections cardiaques du groupe formé par les cardiomyopathies primaires et les cardiomyopathies secondaires, l'acide nucléique (les acides nucléiques) étant le cas échéant formulé avec des excipients ou des supports pharmaceutiquement acceptables.

16. Utilisation d'un vecteur de transfert génétique qui contient une ou plusieurs séquences d'acides nucléiques qui codent pour une ou protéines S100, un ou plusieurs de leurs mutants qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39 pour fabriquer une préparation pharmaceutique visant à traiter les affections cardiaques du groupe formé par les cardiomyopathies primaires et les cardiomyopathies secondaires, le vecteur étant le cas échéant formulé avec des excipients ou des supports pharmaceutiquement acceptables.

17. Utilisation selon les revendications 14 à 16, **caractérisée en ce que** les cardiomyopathies primaires englobent des cardiomyopathies héréditaires et des cardiomyopathies sur terrain de mutations spontanées.

18. Utilisation selon les revendications 14 à 16, **caractérisée en ce que** les cardiomyopathies secondaires englobent la cardiomyopathie ischémique sur un terrain d'artériosclérose, de cardiomyopathie dilatative sur un terrain d'affection infectieuse ou toxique du myocarde, d'affection cardiaque hypertensive sur terrain d'hypertonie des artères pulmonaires et/ou hypertonie artérielle, d'affections cardiaques structurelles sur terrain de troubles du rythme et d'affections des valves du coeur.

19. Utilisation d'une ou de plusieurs protéines S100, d'un ou de plusieurs de leurs mutants qui accroissent la libération systolique de Ca²⁺ issue du réticulum sarcopmastique et accélèrent une réabsorption de Ca²⁺ dans le réticulum sarcoplasmique, ou d'un ou de plusieurs peptides hydrophobes issu(s) du groupe constitué de peptides selon SEQ ID NO : 36 et 39 pour fabriquer une préparation pharmaceutique traitant l'insuffisance cardiaque.

20. Utilisation d'une ou plusieurs séquences d'acides nucléiques qui codent pour une ou plusieurs protéines S100, un ou plusieurs de leurs mutants qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39 pour fabriquer une préparation pharmaceutique traitant l'insuffisance cardiaque, l'acide nucléique (les acides nucléiques) étant le cas échéant formulé avec des excipients ou des supports pharmaceutiquement acceptables.

21. Utilisation d'un vecteur de transfert génétique codant pour une ou plusieurs protéines S100, un ou plusieurs de leurs mutants qui accroissent la libération systolique de Ca²⁺ issu du réticulum sarcoplastique et accélèrent une réabsorption de Ca²⁺ dans le réticulum sarcoplastique, ou pour un ou plusieurs peptides hydrophobes issu(s) du groupe constitué de peptides selon SEQ ID NO : 36 et 39 pour fabriquer une préparation pharmaceutique traitant l'insuffisance cardiaque, le vecteur étant le cas échéant formulé avec des excipients ou des supports pharmaceutiquement acceptables.

22. Utilisation selon les revendications 19 à 21, **caractérisée en ce que** la protéine S100 est choisie dans le groupe formé par S100β, S100A1, S100A2, S100A4 et S100A6.

23. Utilisation selon la revendication 22, **caractérisée en ce que** S100A1 présente la séquence d'acides aminés apparaissant à la SEQ ID NO :2.

24. Utilisation selon la revendication 21, **caractérisée en ce que** le peptide présente la séquence d'acides aminés apparaissant à la SEQ ID NO : 36.

25. Utilisation selon les revendications 14 à 22, **caractérisée en ce qu'**elle utilise une combinaison d'au moins deux des peptides présents parmi SEQ ID NO : 32, 34, 36, 37, 38 et 39.

26. Utilisation selon les revendications 15 à 22, **caractérisée en ce qu'**on utilise une séquence d'acides nucléiques codant pour S100A1 parmi SEQ ID NO : 1 ou un vecteur de transfert génétique contenant cette séquence.

27. Utilisation selon les revendications 16 à 22, **caractérisée en ce que** les séquences d'acides nucléiques codant pour les peptides sont choisies dans SEQ ID NO : 35 ou les vecteurs de transfert génétique contenant cette séquence.

28. Utilisation selon les revendications 14 à 28, **caractérisé en ce qu'**elle utilise une combinaison d'au moins deux séquences d'acides nucléiques codant pour SEQ ID NO : 32, 34, 36, 37, 38 et 39, de préférence au moins deux des séquences d'acides nucléiques présentes parmi SEQ ID NO : 31, 33, 35 et des séquences codant pour SEQ ID NO : 37, 38 et 39, ou un ou plusieurs vecteurs de transfert génétique contenant cette combinaison.

29. Utilisation selon les revendications 14 à 28, **caractérisée en ce qu'**elle est formulée pour une application orale, intraveineuse, intra-artérielle ou intra-coronaire.

30. Peptide **caractérisé en ce qu'**il est constitué par la séquence présente parmi SEQ ID NO : 34, 36, 38 ou 39.

31. Acide nucléique, **caractérisé en ce qu'**il code pour les peptides selon la revendication 30.

32. Vecteur de transformation génétique selon DSM 12755.

33. Procédé de fabrication d'un vecteur de transfert génétique codant pour une ou plusieurs protéines S100 du groupe constitué par S100A1, S100A2, S100A3, S100A4, S100A5, S100A6, S100A7, S100A8, S100A9, S100A10, S100A11, S100A12, S100A13, pour un ou plusieurs de leurs mutants, présentant une homologie d'au moins 60 %, qui accroissent la libération systolique de Ca²⁺ issu du réticulum sarcoplasmique et accélèrent une réabsorption de Ca²⁺ dans le réticulum sarcoplasmique, ou pour un ou plusieurs peptides hydrophobes issu(s) du groupe constitué de peptides selon SEQ ID NO : 36 et 39, **caractérisé en ce que** l'on clone une séquence d'acide nucléique (ou plusieurs) codant pour la protéine (les protéines), le mutant (les mutants) dans un (ou plusieurs) vecteur(s) adapté(s) à la thérapie génétique.

34. Procédé selon la revendication 33, **caractérisé en ce que** l'on choisit la séquence (les séquences) d'acides nucléiques codante dans le groupe des acides nucléiques présents dans SEQ ID NO : 1 à 26.

35. Procédé de fabrication du vecteur de transfert génétique selon la revendication 32, **caractérisé en ce que** l'on clone l'ADNc présent dans SEQ ID NO : 1 dans le site de clonage multiple du vecteur navette pAD TRACK, que l'on linéarise à l'aide de PME1 et que l'on recombine avec pEASY dans E. coli BJ 5183.


### SEQUENZPROTOKOLL

<110> Katus, Hugo A.
   Remppis, Andrew
<120> Therapie der Herzinsuffizienz
<130> P53202
<140>
   <141>
<150> DE 199 15 485.6
   <151> 1999-04-07
<160> 39
<170> PatentIn Vers. 2.0
<210> 1
   <211> 285
   <212> DNA
   <213> Homo sapiens
<220>
   <221> CDS
   <222> (1)..(285)
<223> S100A1 cDNA
<400> 1
   atg ggc tct gag ctg gag acg gcg atg gag acc ctc atc aac gtg ttc     48
   Met Gly Ser Glu Leu Glu Thr Ala Met Glu Thr Leu Ile Asn Val Phe
     1               5                  10                  15
   cac gcc cac tcg ggc aaa gag ggg gac aag tac aag ctg agc aag aag     96
   His Ala His Ser Gly Lys Glu Gly Asp Lys Tyr Lys Leu Ser Lys Lys
                20                  25                  30
   gag ctg aaa gag ctg ctg cag acg gag ctc tct ggc ttc ctg gat gcc    144
   Glu Leu Lys Glu Leu Leu Gln Thr Glu Leu Ser Gly Phe Leu Asp Ala
            35                  40                  45
   cag aag gat gtg gat gct gtg gac aag gtg atg aag gag cta gac gag    192
   Gln Lys Asp Val Asp Ala Val Asp Lys Val Met Lys Glu Leu Asp Glu
        50                  55                  60
   aat gga gac ggg gag gtg gac ttc cag gag tat gtg gtg ctt gtg gct    240
   Asn Gly Asp Gly Glu Val Asp Phe Gln Glu Tyr Val Val Leu Val Ala
    65                  70                  75                  80
   gct ctc aca gtg gcc tgt aac aat ttc ttc tgg gag aac agt tga        285
   Ala Leu Thr Val Ala Cys Asn Asn Phe Phe Trp Glu Asn Ser
                    85                  90                  95
<210> 2
   <211> 94
   <212> PRT
   <213> Homo sapiens
<223> S100A1
<400> 2
   Met Gly Ser Glu Leu Glu Thr Ala Met Glu Thr Leu Ile Asn Val Phe
   1               5                   10                  15
   His Ala His Ser Gly Lys Glu Gly Asp Lys Tyr Lys Leu Ser Lys Lys
               20                  25                  30
   Glu Leu Lys Glu Leu Leu Gln Thr Glu Leu Ser Gly Phe Leu Asp Ala
           35                  40                  45
   Gln Lys Asp Val Asp Ala Val Asp Lys Val Met Lys Glu Leu Asp Glu
       50                  55                  60
   Asn Gly Asp Gly Glu Val Asp Phe Gln Glu Tyr Val Val Leu Val Ala
   65                  70                  75                  80
   Ala Leu Thr Val Ala Cys Asn Asn Phe Phe Trp Glu Asn Ser
                   85                  90
<210> 3
   <211> 294
   <212> DNA
   <213> Homo sapiens
<220>
   <221> CDS
   <222> (1)..(294)
<223> S100A2 cDNA
<400> 3
   atg tgc agt tct ctg gag cag gcg ctg gct gtg ctg gtc act acc ttc      48
   Met Cys Ser Ser Leu Glu Gln Ala Leu Ala Val Leu Val Thr Thr Phe
   1               5                   10                  15
   cac aag tac tcc tgc caa gag ggc gac aag ttc aag ctg agt aag ggg      96
   His Lys Tyr Ser Cys Gln Glu Gly Asp Lys Phe Lys Leu Ser Lys Gly
               20                  25                  30
   gaa atg aag gaa ctt ctg cac aag gag ctg ccc agc ttt gtg ggg gag      144
   Glu Met Lys Glu Leu Leu His Lys Glu Leu Pro Ser Phe Val Gly Glu
           35                  40                  45
   aaa gtg gat gag gag ggg ctg aag aag ctg atg ggc agc ctg gat gag      192
   Lys Val Asp Glu Glu Gly Leu Lys Lys Leu Met Gly Ser Leu Asp Glu
       50                  55                  60
   aac agt gac cag cag gtg gac ttc cag gag tat gct gtt ttc ctg gca      240
   Asn Ser Asp Gln Gln Val Asp Phe Gln Glu Tyr Ala Val Phe Leu Ala
   65                  70                  75                  80
   ctc atc act gtc atg tgc aat gac ttc ttc cag ggc tgc cca gac cga    288
   Leu Ile Thr Val Met Cys Asn Asp Phe Phe Gln Gly Cys Pro Asp Arg
                   85                  90                  95
   ccc tga                                                            294
   Pro
<210> 4
   <211> 97
   <212> PRT
   <213> Homo sapiens
<223> S100A2
<400> 4
   Met Cys Ser Ser Leu Glu Gln Ala Leu Ala Val Leu Val Thr Thr Phe
   1               5                   10                  15
   His Lys Tyr Ser Cys Gln Glu Gly Asp Lys Phe Lys Leu Ser Lys Gly
               20                  25                  30
   Glu Met Lys Glu Leu Leu His Lys Glu Leu Pro Ser Phe Val Gly Glu
           35                  40                  45
   Lys Val Asp Glu Glu Gly Leu Lys Lys Leu Met Gly Ser Leu Asp Glu
       50                  55                  60
   Asn Ser Asp Gln Gln Val Asp Phe Gln Glu Tyr Ala Val Phe Leu Ala
   65                  70                  75                  80
   Leu Ile Thr Val Met Cys Asn Asp Phe Phe Gln Gly Cys Pro Asp Arg
                   85                  90                  95
   Pro
<210> 5
   <211> 306
   <212> DNA
   <213> Homo sapiens
<220>
   <221> CDS
   <222> (1)..(306)
<223> S100A3 cDNA
<400> 5
   atg gcc agg cct ctg gag cag gcg gta gct gcc atc gtg tgc acc ttc    48
   Met Ala Arg Pro Leu Glu Gln Ala Val Ala Ala Ile Val Cys Thr Phe
    1               5                  10                  15
   cag gaa tac gca ggg cgc tgt ggg gac aaa tac aag ctc tgc cag gcg    96
   Gln Glu Tyr Ala Gly Arg Cys Gly Asp Lys Tyr Lys Leu Cys Gln Ala
               20                  25                  30
   gag ctc aag gag ctg ctg cag aag gag ctg gcc acc tgg acc ccg act   144
   Glu Leu Lys Glu Leu Leu Gln Lys Glu Leu Ala Thr Trp Thr Pro Thr
           35                  40                  45
   gag ttt cgg gaa tgt gac tac aac aaa ttc atg agt gtt ctg gac acc   192
   Glu Phe Arg Glu Cys Asp Tyr Asn Lys Phe Met Ser Val Leu Asp Thr
       50                  55                  60
   aac aag gac tgc gag gtg gac ttt gtg gag tat gtg cgc tca ctt gcc   240
   Asn Lys Asp Cys Glu Val Asp Phe Val Glu Tyr Val Arg Ser Leu Ala
   65                  70                  75                  80
   tgc ctc tgt ctc tac tgc cac gag tac ttc aag gac tgc ccc tca gag   288
   Cys Leu Cys Leu Tyr Cys His Glu Tyr Phe Lys Asp Cys Pro Ser Glu
                   85                  90                  95
   ccc ccc tgc tcc cag tag                                           306
   Pro Pro Cys Ser Gln
               100
<210> 6
   <211> 101
   <212> PRT
   <213> Homo sapiens
<223> S100A3
<400> 6
   Met Ala Arg Pro Leu Glu Gln Ala Val Ala Ala Ile Val Cys Thr Phe
    1               5                  10                  15
   Gln Glu Tyr Ala Gly Arg Cys Gly Asp Lys Tyr Lys Leu Cys Gln Ala
               20                  25                  30
   Glu Leu Lys Glu Leu Leu Gln Lys Glu Leu Ala Thr Trp Thr Pro Thr
           35                  40                  45
   Glu Phe Arg Glu Cys Asp Tyr Asn Lys Phe Met Ser Val Leu Asp Thr
       50                  55                  60
   Asn Lys Asp Cys Glu Val Asp Phe Val Glu Tyr Val Arg Ser Leu Ala
   65                  70                  75                  80
   Cys Leu Cys Leu Tyr Cys His Glu Tyr Phe Lys Asp Cys Pro Ser Glu
                   85                  90                  95
   Pro Pro Cys Ser Gln
               100
<210> 7
   <211> 306
   <212> DNA
   <213> Homo sapiens
<220>
   <221> CDS
   <222> (1)..(306)
<223> S100A4 cDNA
<400> 7
   atg gcg tgc cct ctg gag aag gcc ctg gat gtg atg gtg tcc acc ttc      48
   Met Ala Cys Pro Leu Glu Lys Ala Leu Asp Val Met Val Ser Thr Phe
   1               5                   10                  15
   cac aag tac tcg ggc aaa gag ggt gac aag ttc aag ctc aac aag tca      96
   His Lys Tyr Ser Gly Lys Glu Gly Asp Lys Phe Lys Leu Asn Lys Ser
               20                  25                  30
   gaa cta aag gag ctg ctg acc cgg gag ctg ccc agc ttc ttg ggg aaa     144
   Glu Leu Lys Glu Leu Leu Thr Arg Glu Leu Pro Ser Phe Leu Gly Lys
           35                  40                  45
   agg aca gat gaa gct gct ttc cag aag ctg atg agc aac ttg gac agc     192
   Arg Thr Asp Glu Ala Ala Phe Gln Lys Leu Met Ser Asn Leu Asp Ser
       50                  55                  60
   aac agg gac aac gag gtg gac ttc caa gag tac tgt gtc ttc ctg tcc     240
   Asn Arg Asp Asn Glu Val Asp Phe Gln Glu Tyr Cys Val Phe Leu Ser
   65                  70                  75                  80
   tgc atc gcc atg atg tgt aac gaa ttc ttt gaa ggc ttc cca gat aag     288
   Cys Ile Ala Met Met Cys Asn Glu Phe Phe Glu Gly Phe Pro Asp Lys
                   85                  90                  95
   cag ccc agg aag aaa tga                                             306
   Gln Pro Arg Lys Lys
               100
<210> 8
   <211> 101
   <212> PRT
   <213> Homo sapiens
<223> S100A4
<400> 8
   Met Ala Cys Pro Leu Glu Lys Ala Leu Asp Val Met Val Ser Thr Phe
    1               5                  10                  15
   His Lys Tyr Ser Gly Lys Glu Gly Asp Lys Phe Lys Leu Asn Lys Ser
               20                  25                  30
   Glu Leu Lys Glu Leu Leu Thr Arg Glu Leu Pro Ser Phe Leu Gly Lys
           35                  40                  45
   Arg Thr Asp Glu Ala Ala Phe Gln Lys Leu Met Ser Asn Leu Asp Ser
       50                  55                  60
   Asn Arg Asp Asn Glu Val Asp Phe Gln Glu Tyr Cys Val Phe Leu Ser
   65                  70                  75                  80
   Cys Ile Ala Met Met Cys Asn Glu Phe Phe Glu Gly Phe Pro Asp Lys
                   85                  90                  95
   Gln Pro Arg Lys Lys
               100
<210> 9
   <211> 333
   <212> DNA
   <213> Homo sapiens
<220>
   <221> CDS
   <222> (1)..(333)
<223> S100A5 cDNA
<400> 9
   atg cct gct gct tgg att ctc tgg gct cac tcc cac agt gag ctg cac     48
   Met Pro Ala Ala Trp Ile Leu Trp Ala His Ser His Ser Glu Leu His
    1               5                  10                  15
   act gtg atg gag act cct ctg gag aag gcc ctg acc act atg gtg acc     96
   Thr Val Met Glu Thr Pro Leu Glu Lys Ala Leu Thr Thr Met Val Thr
               20                  25                  30
   acg ttt cac aaa tat tcg ggg aga gag ggt agc aaa ctg acc ctg agt    144
   Thr Phe His Lys Tyr Ser Gly Arg Glu Gly Ser Lys Leu Thr Leu Ser
           35                  40                  45
   agg aag gaa ctc aag gag ctg atc aag aaa gag ctg tgt ctt ggg gag    192
   Arg Lys Glu Leu Lys Glu Leu Ile Lys Lys Glu Leu Cys Leu Gly Glu
       50                  55                  60
   atg aag gag agc agc atc gat gac ttg atg aag agc ctg gac aag aac    240
   Met Lys Glu Ser Ser Ile Asp Asp Leu Met Lys Ser Leu Asp Lys Asn
   65                  70                  75                  80
   agc gac cag gag atc gac ttc aag gag tac tcg gtg ttc ctg acc atg    288
   Ser Asp Gln Glu Ile Asp Phe Lys Glu Tyr Ser Val Phe Leu Thr Met
                   85                  90                  95
   ctg tgc atg gcc tac aac gac ttc ttt cta gag gac aac aag tga        333
   Leu Cys Met Ala Tyr Asn Asp Phe Phe Leu Glu Asp Asn Lys
               100                 105                 110
<210> 10
   <211> 110
   <212> PRT
   <213> Homo sapiens
<223> S100A5
<400> 10
   Met Pro Ala Ala Trp Ile Leu Trp Ala His Ser His Ser Glu Leu His
    1               5                  10                  15
   Thr Val Met Glu Thr Pro Leu Glu Lys Ala Leu Thr Thr Met Val Thr
               20                  25                  30
   Thr Phe His Lys Tyr Ser Gly Arg Glu Gly Ser Lys Leu Thr Leu Ser
           35                  40                  45
   Arg Lys Glu Leu Lys Glu Leu Ile Lys Lys Glu Leu Cys Leu Gly Glu
       50                  55                  60
   Met Lys Glu Ser Ser Ile Asp Asp Leu Met Lys Ser Leu Asp Lys Asn
   65                  70                  75                  80
   Ser Asp Gln Glu Ile Asp Phe Lys Glu Tyr Ser Val Phe Leu Thr Met
                   85                  90                  95
   Leu Cys Met Ala Tyr Asn Asp Phe Phe Leu Glu Asp Asn Lys
               100                 105                 110
<210> 11
   <211> 273
   <212> DNA
   <213> Homo sapiens
<220>
   <221> CDS
   <222> (1)..(273)
<223> S100A6 cDNA
<400> 11
   atg gca tgc ccc ctg gat cag gcc att ggc ctc ctc gtg gcc atc ttc      48
   Met Ala Cys Pro Leu Asp Gln Ala Ile Gly Leu Leu Val Ala Ile Phe
     1               5                  10                  15
   cac aag tac tcc ggc agg gag ggt gac aag cac acc ctg agc aag aag      96
   His Lys Tyr Ser Gly Arg Glu Gly Asp Lys His Thr Leu Ser Lys Lys
                20                  25                  30
   gag ctg aag gag ctg atc cag aag gag ctc acc att ggc tcg aag ctg     144
   Glu Leu Lys Glu Leu Ile Gln Lys Glu Leu Thr Ile Gly Ser Lys Leu
            35                  40                  45
   cag gat gct gaa att gca agg ctg atg gaa gac ttg gac cgg aac aag     192
   Gln Asp Ala Glu Ile Ala Arg Leu Met Glu Asp Leu Asp Arg Asn Lys
        50                  55                  60
   gac cag gag gtg aac ttc cag gag tat gtc acc ttc ctg ggg gcc ttg     240
   Asp Gln Glu Val Asn Phe Gln Glu Tyr Val Thr Phe Leu Gly Ala Leu
    65                  70                  75                  80
   gct ttg atc tac aat gaa gcc ctc aag ggc tga                         273
   Ala Leu Ile Tyr Asn Glu Ala Leu Lys Gly
                    85                  90
<210> 12
   <211> 90
   <212> PRT
   <213> Homo sapiens
<223> S100A6
<400> 12
   Met Ala Cys Pro Leu Asp Gln Ala Ile Gly Leu Leu Val Ala Ile Phe
     1               5                  10                  15
   His Lys Tyr Ser Gly Arg Glu Gly Asp Lys His Thr Leu Ser Lys Lys
                20                  25                  30
   Glu Leu Lys Glu Leu Ile Gln Lys Glu Leu Thr Ile Gly Ser Lys Leu
           35                  40                  45
   Gln Asp Ala Glu Ile Ala Arg Leu Met Glu Asp Leu Asp Arg Asn Lys
       50                  55                  60
   Asp Gln Glu Val Asn Phe Gln Glu Tyr Val Thr Phe Leu Gly Ala Leu
   65                  70                  75                  80
   Ala Leu Ile Tyr Asn Glu Ala Leu Lys Gly
                   85                  90
<210> 13
   <211> 306
   <212> DNA
   <213> Homo sapiens
<220>
   <221> CDS
   <222> (1)..(306)
<223> S100A7 cDNA
<400> 13
   atg agc aac act caa gct gag agg tcc ata ata ggc atg atc gac atg      48
   Met Ser Asn Thr Gln Ala Glu Arg Ser Ile Ile Gly Met Ile Asp Met
    1               5                  10                  15
   ttt cac aaa tac acc aga cgt gat gac aag att gac aag cca agc ctg      96
   Phe His Lys Tyr Thr Arg Arg Asp Asp Lys Ile Asp Lys Pro Ser Leu
               20                  25                  30
   ctg acg atg atg aag gag aac ttc ccc aac ttc ctt agt gcc tgt gac     144
   Leu Thr Met Met Lys Glu Asn Phe Pro Asn Phe Leu Ser Ala Cys Asp
           35                  40                  45
   aaa aag ggc aca aat tac ctc gcc gac gtc ttt gag aaa aag gac aag     192
   Lys Lys Gly Thr Asn Tyr Leu Ala Asp Val Phe Glu Lys Lys Asp Lys
       50                  55                  60
   aat gag gat aag aag att gat ttt tct gag ttt ctg tcc ttg ctg gga     240
   Asn Glu Asp Lys Lys Ile Asp Phe Ser Glu Phe Leu Ser Leu Leu Gly
   65                  70                  75                  80
   gac ata gcc aca gac tac cac aag cag agc cat gga gca gcg ccc tgt     288
   Asp Ile Ala Thr Asp Tyr His Lys Gln Ser His Gly Ala Ala Pro Cys
                   85                  90                  95
   tcc ggg ggc agc cag tga                                             306
   Ser Gly Gly Ser Gln
               100
<210> 14
   <211> 101
   <212> PRT
   <213> Homo sapiens
<223> S100A7
<400> 14
   Met Ser Asn Thr Gln Ala Glu Arg Ser Ile Ile Gly Met Ile Asp Met
   1               5                   10                  15
   Phe His Lys Tyr Thr Arg Arg Asp Asp Lys Ile Asp Lys Pro Ser Leu
               20                  25                  30
   Leu Thr Met Met Lys Glu Asn Phe Pro Asn Phe Leu Ser Ala Cys Asp
           35                  40                  45
   Lys Lys Gly Thr Asn Tyr Leu Ala Asp Val Phe Glu Lys Lys Asp Lys
       50                  55                  60
   Asn Glu Asp Lys Lys Ile Asp Phe Ser Glu Phe Leu Ser Leu Leu Gly
   65                  70                  75                  80
   Asp Ile Ala Thr Asp Tyr His Lys Gln Ser His Gly Ala Ala Pro Cys
                   85                  90                  95
   Ser Gly Gly Ser Gln
               100
<210> 15
   <211> 282
   <212> DNA
   <213> Homo sapiens
<220>
   <221> CDS
   <222> (1)..(282)
<223> S100A8 cDNA
<400> 15
   atg ttg acc gag ctg gag aaa gcc ttg aac tct atc atc gac gtc tac      48
   Met Leu Thr Glu Leu Glu Lys Ala Leu Asn Ser Ile Ile Asp Val Tyr
   1               5                   10                  15
   cac aag tac tcc ctg ata aag ggg aat ttc cat gcc gtc tac agg gat      96
   His Lys Tyr Ser Leu Ile Lys Gly Asn Phe His Ala Val Tyr Arg Asp
               20                  25                  30
   gac ctg aag aaa ttg cta gag acc gag tgt cct cag tat atc agg aaa     144
   Asp Leu Lys Lys Leu Leu Glu Thr Glu Cys Pro Gln Tyr Ile Arg Lys
           35                  40                  45
   aag ggt gca gac gtc tgg ttc aaa gag ttg gat atc aac act gat ggt     192
   Lys Gly Ala Asp Val Trp Phe Lys Glu Leu Asp Ile Asn Thr Asp Gly
       50                  55                  60
   gca gtt aac ttc cag gag ttc ctc att ctg gtg ata aag atg ggc gtg     240
   Ala Val Asn Phe Gln Glu Phe Leu Ile Leu Val Ile Lys Met Gly Val
   65                  70                  75                  80
   gca gcc cac aaa aaa agc cat gaa gaa agc cac aaa gag tag             282
   Ala Ala His Lys Lys Ser His Glu Glu Ser His Lys Glu
                   85                  90
<210> 16
   <211> 93
   <212> PRT
   <213> Homo sapiens
<223> S100A8
<400> 16
   Met Leu Thr Glu Leu Glu Lys Ala Leu Asn Ser Ile Ile Asp Val Tyr
     1               5                  10                  15
   His Lys Tyr Ser Leu Ile Lys Gly Asn Phe His Ala Val Tyr Arg Asp
                20                  25                  30
   Asp Leu Lys Lys Leu Leu Glu Thr Glu Cys Pro Gln Tyr Ile Arg Lys
            35                  40                  45
   Lys Gly Ala Asp Val Trp Phe Lys Glu Leu Asp Ile Asn Thr Asp Gly
        50                  55                  60
   Ala Val Asn Phe Gln Glu Phe Leu Ile Leu Val Ile Lys Met Gly Val
    65                  70                  75                  80
   Ala Ala His Lys Lys Ser His Glu Glu Ser His Lys Glu
                    85                  90
<210> 17
   <211> 345
   <212> DNA
   <213> Homo sapiens
<220>
   <221> CDS
   <222> (1)..(345)
<223> S100A9 cDNA
<400> 17
   atg act tgc aaa atg tcg cag ctg gaa cgc aac ata gag acc atc atc    48
   Met Thr Cys Lys Met Ser Gln Leu Glu Arg Asn Ile Glu Thr Ile Ile
     1               5                  10                  15
   aac acc ttc cac caa tac tct gtg aag ctg ggg cac cca gac acc ctg    96
   Asn Thr Phe His Gln Tyr Ser Val Lys Leu Gly His Pro Asp Thr Leu
                20                  25                  30
   aac cag ggg gaa ttc aaa gag ctg gtg cga aaa gat ctg caa aat ttt    144
   Asn Gln Gly Glu Phe Lys Glu Leu Val Arg Lys Asp Leu Gln Asn Phe
            35                  40                  45
   ctc aag aag gag aat aag aat gaa aag gtc ata gaa cac atc atg gag    192
   Leu Lys Lys Glu Asn Lys Asn Glu Lys Val Ile Glu His Ile Met Glu
        50                  55                  60
   gac ctg gac aca aat gca gac aag cag ctg agc ttc gag gag ttc atc    240
   Asp Leu Asp Thr Asn Ala Asp Lys Gln Leu Ser Phe Glu Glu Phe Ile
    65                  70                  75                  80
   atg ctg atg gcg agg cta acc tgg gcc tcc cac gag aag atg cac gag     288
   Met Leu Met Ala Arg Leu Thr Trp Ala Ser His Glu Lys Met His Glu
                   85                  90                  95
   ggt gac gag ggc cct ggc cac cac cat aag cca ggc ctc ggg gag ggc     336
   Gly Asp Glu Gly Pro Gly His His His Lys Pro Gly Leu Gly Glu Gly
               100                 105                 110
   acc ccc taa                                                         345
   Thr Pro
           115
<210> 18
   <211> 114
   <212> PRT
   <213> Homo sapiens
<223> S100A9
<400> 18
   Met Thr Cys Lys Met Ser Gln Leu Glu Arg Asn Ile Glu Thr Ile Ile
    1               5                  10                  15
   Asn Thr Phe His Gln Tyr Ser Val Lys Leu Gly His Pro Asp Thr Leu
               20                  25                  30
   Asn Gln Gly Glu Phe Lys Glu Leu Val Arg Lys Asp Leu Gln Asn Phe
           35                  40                  45
   Leu Lys Lys Glu Asn Lys Asn Glu Lys Val Ile Glu His Ile Met Glu
       50                  55                  60
   Asp Leu Asp Thr Asn Ala Asp Lys Gln Leu Ser Phe Glu Glu Phe Ile
   65                  70                  75                  80
   Met Leu Met Ala Arg Leu Thr Trp Ala Ser His Glu Lys Met His Glu
                   85                  90                  95
   Gly Asp Glu Gly Pro Gly His His His Lys Pro Gly Leu Gly Glu Gly
               100                 105                 110
   Thr Pro
<210> 19
   <211> 294
   <212> DNA
   <213> Homo sapiens
<220>
   <221> CDS
   <222> (1)..(294)
<223> S100A10 cDNA
<400> 19
   atg cca tct caa atg gaa cac gcc atg gaa acc atg atg ttt aca ttt   48
   Met Pro Ser Gln Met Glu His Ala Met Glu Thr Met Met Phe Thr Phe
    1               5                   10                  15
   cac aaa ttc gct ggg gat aaa ggc tac tta aca aag gag gac ctg aga   96
   His Lys Phe Ala Gly Asp Lys Gly Tyr Leu Thr Lys Glu Asp Leu Arg
               20                  25                  30
   gta ctc atg gaa aag gag ttc cct gga ttt ttg gaa aat caa aaa gac   144
   Val Leu Met Glu Lys Glu Phe Pro Gly Phe Leu Glu Asn Gln Lys Asp
           35                  40                  45
   cct ctg gct gtg gac aaa ata atg aag gac ctg gac cag tgt aga gat   192
   Pro Leu Ala Val Asp Lys Ile Met Lys Asp Leu Asp Gln Cys Arg Asp
       50                  55                  60
   ggc aaa gtg ggc ttc cag agc ttc ttt tcc cta att gcg ggc ctc acc   240
   Gly Lys Val Gly Phe Gln Ser Phe Phe Ser Leu Ile Ala Gly Leu Thr
   65                  70                  75                  80
   att gca tgc aat gac tat ttt gta gta cac atg aag cag aag gga aag   288
   Ile Ala Cys Asn Asp Tyr Phe Val Val His Met Lys Gln Lys Gly Lys
                   85                  90                  95
   aag tag                                                           294
   Lys
<210> 20
   <211> 97
   <212> PRT
   <213> Homo sapiens
<223> S100A10
<400> 20
   Met Pro Ser Gln Met Glu His Ala Met Glu Thr Met Met Phe Thr Phe
    1               5                   10                  15
   His Lys Phe Ala Gly Asp Lys Gly Tyr Leu Thr Lys Glu Asp Leu Arg
               20                  25                  30
   Val Leu Met Glu Lys Glu Phe Pro Gly Phe Leu Glu Asn Gln Lys Asp
           35                  40                  45
   Pro Leu Ala Val Asp Lys Ile Met Lys Asp Leu Asp Gln Cys Arg Asp
       50                  55                  60
   Gly Lys Val Gly Phe Gln Ser Phe Phe Ser Leu Ile Ala Gly Leu Thr
   65                  70                  75                  80
   Ile Ala Cys Asn Asp Tyr Phe Val Val His Met Lys Gln Lys Gly Lys
                   85                  90                  95
   Lys
<210> 21
   <211> 318
   <212> DNA
   <213> Homo sapiens
<220>
   <221> CDS
   <222> (1)..(318)
<223> S100A11 cDNA
<400> 21
   atg gca aaa atc tcc agc cct aca gag act gag cgg tgc atc gag tcc      48
   Met Ala Lys Ile Ser Ser Pro Thr Glu Thr Glu Arg Cys Ile Glu Ser
    1               5                  10                  15
   ctg att gct gtc ttc cag aag tat gct gga aag gat ggt tat aac tac      96
   Leu Ile Ala Val Phe Gln Lys Tyr Ala Gly Lys Asp Gly Tyr Asn Tyr
               20                  25                  30
   act ctc tcc aag aca gag ttc cta agc ttc atg aat aca gaa cta gct     144
   Thr Leu Ser Lys Thr Glu Phe Leu Ser Phe Met Asn Thr Glu Leu Ala
           35                  40                  45
   gcc ttc aca aag aac cag aag gac cct ggt gtc ctt gac cgc atg atg     192
   Ala Phe Thr Lys Asn Gln Lys Asp Pro Gly Val Leu Asp Arg Met Met
       50                  55                  60
   aag aaa ctg gac acc aac agt gat ggt cag cta gat ttc tca gaa ttt     240
   Lys Lys Leu Asp Thr Asn Ser Asp Gly Gln Leu Asp Phe Ser Glu Phe
   65                  70                  75                  80
   ctt aat ctg att ggt ggc cta gct atg gct tgc cat gac tcc ttc ctc     288
   Leu Asn Leu Ile Gly Gly Leu Ala Met Ala Cys His Asp Ser Phe Leu
                   85                  90                  95
   aag gct gtc cct tcc cag aag cgg acc tga                             318
   Lys Ala Val Pro Ser Gln Lys Arg Thr
               100                 105
<210> 22
   <211> 105
   <212> PRT
   <213> Homo sapiens
<223> S100A11
<400> 22
   Met Ala Lys Ile Ser Ser Pro Thr Glu Thr Glu Arg Cys Ile Glu Ser
   1               5                   10                  15
   Leu Ile Ala Val Phe Gln Lys Tyr Ala Gly Lys Asp Gly Tyr Asn Tyr
               20                  25                  30
   Thr Leu Ser Lys Thr Glu Phe Leu Ser Phe Met Asn Thr Glu Leu Ala
           35                  40                  45
   Ala Phe Thr Lys Asn Gln Lys Asp Pro Gly Val Leu Asp Arg Met Met
       50                  55                  60
   Lys Lys Leu Asp Thr Asn Ser Asp Gly Gln Leu Asp Phe Ser Glu Phe
   65                  70                  75                  80
   Leu Asn Leu Ile Gly Gly Leu Ala Met Ala Cys His Asp Ser Phe Leu
                   85                  90                  95
   Lys Ala Val Pro Ser Gln Lys Arg Thr
               100                 105
<210> 23
   <211> 279
   <212> DNA
   <213> Homo sapiens
<220>
   <221> CDS
   <222> (1)..(279)
<223> S100A12 cDNA
<400> 23
   atg aca aaa ctt gaa gag cat ctg gag gga att gtc aat atc ttc cac    48
   Met Thr Lys Leu Glu Glu His Leu Glu Gly Ile Val Asn Ile Phe His
   1               5                   10                  15
   caa tac tca gtt cgg aag ggg cat ttt gac acc ctc tct aag ggt gag    96
   Gln Tyr Ser Val Arg Lys Gly His Phe Asp Thr Leu Ser Lys Gly Glu
               20                  25                  30
   ctg aag cag ctg ctt aca aag gag ctt gca aac acc atc aag aat atc    144
   Leu Lys Gln Leu Leu Thr Lys Glu Leu Ala Asn Thr Ile Lys Asn Ile
           35                  40                  45
   aaa gat aaa gct gtc att gat gaa ata ttc caa ggc ctg gat gct aat    192
   Lys Asp Lys Ala Val Ile Asp Glu Ile Phe Gln Gly Leu Asp Ala Asn
       50                  55                  60
   caa gat gaa cag gtc gac ttt caa gaa ttc ata tcc ctg gta gcc att    240
   Gln Asp Glu Gln Val Asp Phe Gln Glu Phe Ile Ser Leu Val Ala Ile
   65                  70                  75                  80
   gcg ctg aag gct gcc cat tac cac acc cac aaa gag tag                279
   Ala Leu Lys Ala Ala His Tyr His Thr His Lys Glu
                   85                  90
<210> 24
   <211> 92
   <212> PRT
   <213> Homo sapiens
<223> S100A12
<400> 24
   Met Thr Lys Leu Glu Glu His Leu Glu Gly Ile Val Asn Ile Phe His
   1               5                   10                  15
   Gln Tyr Ser Val Arg Lys Gly His Phe Asp Thr Leu Ser Lys Gly Glu
               20                  25                  30
   Leu Lys Gln Leu Leu Thr Lys Glu Leu Ala Asn Thr Ile Lys Asn Ile
           35                  40                  45
   Lys Asp Lys Ala Val Ile Asp Glu Ile Phe Gln Gly Leu Asp Ala Asn
       50                  55                  60
   Gln Asp Glu Gln Val Asp Phe Gln Glu Phe Ile Ser Leu Val Ala Ile
   65                  70                  75                  80
   Ala Leu Lys Ala Ala His Tyr His Thr His Lys Glu
                   85                  90
<210> 25
   <211> 297
   <212> DNA
   <213> Homo sapiens
<220>
   <221> CDS
   <222> (1)..(297)
<223> S100A13 cDNA
<400> 25
   atg gca gca gaa cca ctg aca gag cta gag gag tcc att gag acc gtg    48
   Met Ala Ala Glu Pro Leu Thr Glu Leu Glu Glu Ser Ile Glu Thr Val
     1               5                  10                  15
   gtc acc acc ttc ttc acc ttt gca agg cag gag ggc cgg aag gat agc    96
   Val Thr Thr Phe Phe Thr Phe Ala Arg Gln Glu Gly Arg Lys Asp Ser
               20                  25                  30
   ctc agc gtc aac gag ttc aaa gag ctg gtt acc cag cag ttg ccc cat   144
   Leu Ser Val Asn Glu Phe Lys Glu Leu Val Thr Gln Gln Leu Pro His
           35                  40                  45
   ctg ctc aag gat gtg ggc tct ctt gat gag aag atg aag agc ttg gat   192
   Leu Leu Lys Asp Val Gly Ser Leu Asp Glu Lys Met Lys Ser Leu Asp
       50                  55                  60
   gtg aat cag gac tcg gag ctc aag ttc aat gag tac tgg aga ttg att   240
   Val Asn Gln Asp Ser Glu Leu Lys Phe Asn Glu Tyr Trp Arg Leu Ile
    65                  70                  75                  80
   ggg gag ctg gcc aag gaa atc agg aag aag aaa gac ctg aag atc agg   288
   Gly Glu Leu Ala Lys Glu Ile Arg Lys Lys Lys Asp Leu Lys Ile Arg
                    85                  90                  95
   aag aag taa                                                       297
   Lys Lys
<210> 26
   <211> 98
   <212> PRT
   <213> Homo sapiens
<223> S100A13
<400> 26
   Met Ala Ala Glu Pro Leu Thr Glu Leu Glu Glu Ser Ile Glu Thr Val
     1               5                  10                  15
   Val Thr Thr Phe Phe Thr Phe Ala Arg Gln Glu Gly Arg Lys Asp Ser
               20                  25                  30
   Leu Ser Val Asn Glu Phe Lys Glu Leu Val Thr Gln Gln Leu Pro His
           35                  40                  45
   Leu Leu Lys Asp Val Gly Ser Leu Asp Glu Lys Met Lys Ser Leu Asp
       50                  55                  60
   Val Asn Gln Asp Ser Glu Leu Lys Phe Asn Glu Tyr Trp Arg Leu Ile
    65                  70                  75                  80
   Gly Glu Leu Ala Lys Glu Ile Arg Lys Lys Lys Asp Leu Lys Ile Arg
                    85                  90                  95
   Lys Lys
<210> 27
   <211> 288
   <212> DNA
   <213> Homo sapiens
<220>
   <221> CDS
   <222> (1)..(288)
<223> S100B cDNA
<400> 27
   atg acg gaa cta gag aca gcc atg ggc atg atc ata gac gtc ttt tcc    48
   Met Thr Glu Leu Glu Thr Ala Met Gly Met Ile Ile Asp Val Phe Ser
     1               5                  10                  15
   cga tat tcg ggc agc gag ggc agc acg cag acc ctg acc aag ggg gag    96
   Arg Tyr Ser Gly Ser Glu Gly Ser Thr Gln Thr Leu Thr Lys Gly Glu
                20                  25                  30
   ctc aag gtg ctg atg gag aag gag cta cca ggc ttc ctg cag agt gga    144
   Leu Lys Val Leu Met Glu Lys Glu Leu Pro Gly Phe Leu Gln Ser Gly
            35                  40                  45
   aaa gac aag gat gcc gtg gat aaa ttg ctc aag gac ctg gac gcc aat    192
   Lys Asp Lys Asp Ala Val Asp Lys Leu Leu Lys Asp Leu Asp Ala Asn
        50                  55                  60
   gga gat gcc cag gtg gac ttc agt gag ttc atc gtg ttc gtg gct gca    240
   Gly Asp Ala Gln Val Asp Phe Ser Glu Phe Ile Val Phe Val Ala Ala
    65                  70                  75                  80
   atc acg tct gcc tgt cac aag tac ttt gag aag gca gga ctc aaa tga    288
   Ile Thr Ser Ala Cys His Lys Tyr Phe Glu Lys Ala Gly Leu Lys
                    85                  90                  95
<210> 28
   <211> 95
   <212> PRT
   <213> Homo sapiens
<223> S100B
<400> 28
   Met Thr Glu Leu Glu Thr Ala Met Gly Met Ile Ile Asp Val Phe Ser
     1               5                  10                  15
   Arg Tyr Ser Gly Ser Glu Gly Ser Thr Gln Thr Leu Thr Lys Gly Glu
                20                  25                  30
   Leu Lys Val Leu Met Glu Lys Glu Leu Pro Gly Phe Leu Gln Ser Gly
            35                  40                  45
   Lys Asp Lys Asp Ala Val Asp Lys Leu Leu Lys Asp Leu Asp Ala Asn
        50                  55                  60
   Gly Asp Ala Gln Val Asp Phe Ser Glu Phe Ile Val Phe Val Ala Ala
    65                  70                  75                  80
   Ile Thr Ser Ala Cys His Lys Tyr Phe Glu Lys Ala Gly Leu Lys
                    85                  90                  95
<210> 29
   <211> 288
   <212> DNA
   <213> Homo sapiens
<220>
   <221> CDS
   <222> (1)..(288)
<223> S100P cDNA
<400> 29
   atg acg gaa cta gag aca gcc atg ggc atg atc ata gac gtc ttt tcc     48
   Met Thr Glu Leu Glu Thr Ala Met Gly Met Ile Ile Asp Val Phe Ser
     1               5                  10                  15
   cga tat tcg ggc agc gag ggc agc acg cag acc ctg acc aag ggg gag     96
   Arg Tyr Ser Gly Ser Glu Gly Ser Thr Gln Thr Leu Thr Lys Gly Glu
                20                  25                  30
   ctc aag gtg ctg atg gag aag gag cta cca ggc ttc ctg cag agt gga    144
   Leu Lys Val Leu Met Glu Lys Glu Leu Pro Gly Phe Leu Gln Ser Gly
            35                  40                  45
   aaa gac aag gat gcc gtg gat aaa ttg ctc aag gac ctg gac gcc aat    192
   Lys Asp Lys Asp Ala Val Asp Lys Leu Leu Lys Asp Leu Asp Ala Asn
        50                  55                  60
   gga gat gcc cag gtg gac ttc agt gag ttc atc gtg ttc gtg gct gca    240
   Gly Asp Ala Gln Val Asp Phe Ser Glu Phe Ile Val Phe Val Ala Ala
    65                  70                  75                  80
   atc acg tct gcc tgt cac aag tac ttt gag aag gca gga ctc aaa tga    288
   Ile Thr Ser Ala Cys His Lys Tyr Phe Glu Lys Ala Gly Leu Lys
                    85                  90                  95
<210> 30
   <211> 95
   <212> PRT
   <213> Homo sapiens
<223> S100P
<400> 30
   Met Thr Glu Leu Glu Thr Ala Met Gly Met Ile Ile Asp Val Phe Ser
    1               5                  10                  15
   Arg Tyr Ser Gly Ser Glu Gly Ser Thr Gln Thr Leu Thr Lys Gly Glu
               20                  25                  30
   Leu Lys Val Leu Met Glu Lys Glu Leu Pro Gly Phe Leu Gln Ser Gly
           35                  40                  45
   Lys Asp Lys Asp Ala Val Asp Lys Leu Leu Lys Asp Leu Asp Ala Asn
       50                  55                  60
   Gly Asp Ala Gln Val Asp Phe Ser Glu Phe Ile Val Phe Val Ala Ala
   65                  70                  75                  80
   Ile Thr Ser Ala Cys His Lys Tyr Phe Glu Lys Ala Gly Leu Lys
                   85                  90                  95
<210> 31
   <211> 45
   <212> DNA
   <213> Homo sapiens
<220>
   <221> CDS
   <222> (1)..(45)
<223> S100A1 cDNA [N-terminal]
<400> 31
   ggc tct gag ctg gag acg gcg atg gag acc ctc atc aac gtg ttc        45
   Gly Ser Glu Leu Glu Thr Ala Met Glu Thr Leu Ile Asn Val Phe
    1               5                  10                  15
<210> 32
   <211> 15
   <212> PRT
   <213> Homo sapiens
<223> S100A1 [N-terminal]
<400> 32
   Gly Ser Glu Leu Glu Thr Ala Met Glu Thr Leu Ile Asn Val Phe
    1               5                  10                  15
<210> 33
   <211> 39
   <212> DNA
   <213> Homo sapiens
<220>
   <221> CDS
   <222> (1)..(39)
<223> S100A1 cDNA [hinge-region]
<400> 33
   ctc tct ggc ttc ctg gat gcc cag aag gat gtg gat gct                39
   Leu Ser Gly Phe Leu Asp Ala Gln Lys Asp Val Asp Ala
     1               5                  10
<210> 34
   <211> 13
   <212> PRT
   <213> Homo sapiens
<400> 34 <223> S100A1 [hinge-region]
<210> 35
   <211> 33
   <212> DNA
   <213> Homo sapiens
<220>
   <221> CDS
   <222> (1)..(33)
<223> S100A1 cDNA [C-terminal]
<400> 35
   tat gtg gtg ctt gtg gct gct ctc aca gtg gcc                33
   Tyr Val Val Leu Val Ala Ala Leu Thr Val Ala
     1               5                  10
<210> 36
   <211> 11
   <212> PRT
   <213> Homo sapiens
<223> S100A1 [C-terminal]
<400> 36
   Tyr Val Val Leu Val Ala Ala Leu Thr Val Ala
     1               5                  10
<210> 37
   <211> 17
   <212> PRT
   <213> Homo sapiens
<223> S100A1 [N-terminal, modifiziert]
<400> 37
   Gly Ser Glu Leu Glu Thr Ala Met Glu Thr Leu Ile Asn Val Phe His
    1               5                  10                  15
   Ala
<210> 38
   <211> 13
   <212> PRT
   <213> Homo sapiens
<223> S100A1 [hinge-region, modifiziert]
<400> 38
   Leu Ser Gly Phe Leu Asp Ala Gln Lys Asp Ala Asp Ala
    1               5                  10
<210> 39
   <211> 17
   <212> PRT
   <213> Homo sapiens
<223> S100A1 [C-terminal, modifiziert]
<400> 39
   Asp Lys Asp Asp Pro Pro Tyr Val Val Leu Val Ala Ala Leu Thr Val
    1               5                  10                  15
   Ala